# EUROPEAN PATENT APPLICATION

(11) **EP 4 810 064 A1**
(43) Date of publication of application: **23.09.2026**
(21) Application number: 24890734.7
(22) Date of filing: 13.11.2024
(51) Int. Cl.: A61K 47/68, A61P 35/00

(54) **METHOD FOR TREATING CANCER BY ANTI-B7H3 ANTIBODY-DRUG CONJUGATE**

(30) Priority: 13.11.2023 CN 202311501263; 07.08.2024 CN 202411076978
(71) Applicant: Medilink Therapeutics (Suzhou) Co., Ltd., Suzhou, Jiangsu 215000 (CN)
(72) Inventor: XUE, Tongtong, Suzhou, Jiangsu 215000 (CN); LIAN, Wei, Suzhou, Jiangsu 215000 (CN); CAI, Jiaqiang, Suzhou, Jiangsu 215000 (CN); CHIN, Steve, Suzhou, Jiangsu 215000 (CN); WANG, Ruihua, Suzhou, Jiangsu 215000 (CN); ZHANG, Xian, Suzhou, Jiangsu 215000 (CN); XIAO, Liang, Suzhou, Jiangsu 215000 (CN)
(74) Representative: Mewburn Ellis LLP
(86) International application number: PCT/CN2024/131843
(87) International publication number: WO 2025/103379

(57) **Abstract**

A method or use of administering an anti-B7H3 antibody-drug conjugate in combination with other therapeutic agents for treating cancer. The other therapeutic agents are selected from anti-PD-1 antibodies or antigen-binding fragments or/and chemotherapeutic drugs thereof. Also provided is a use of the combination in a pharmaceutical composition and a pharmaceutical kit for treating cancer.

## Description

The present application claims priority to the Chinese patent application 2023115012636 filed on November 13, 2023 and the Chinese patent application 2024110769786 filed on August 7, 2024. The above-mentioned Chinese patent applications are incorporated herein by reference in their entirety.

### TECHNICAL FIELD

The present invention pertains to the technical field of molecular immunology and tumour treatment. The present invention relates to a method or use related to administering an anti-B7H3 antibody-drug conjugate in combination with an additional therapeutic agent to treat cancer; the additional therapeutic agent is selected from an anti-PD-1 antibody or antigen-binding fragment thereof or/and a chemotherapeutic drug. The present invention further relates to the use of the combination in a pharmaceutical composition and a pharmaceutical kit used in the treatment of cancer.

### BACKGROUND ART

Malignant tumours have become a major global public health problem, resulting in nearly 10 million deaths in 2020. The number of cancer patients and the number of deaths due to cancer have continued to rise globally, resulting in the continuous expansion of the overall cancer treatment market and an increasing demand for new therapeutic drugs and treatment modalities.

Monoclonal antibody drugs have the advantages of strong targeting, high specificity and a low incidence of adverse reactions, and antibody-drug conjugates (ADC) are representative products among those drugs. Since ADC possesses both the efficacy of small molecule drugs and the targeting properties of antibody drugs, it can reduce the toxicity of cytotoxic drugs while enhancing therapeutic effects. At present, 15 ADC drugs have been approved for marketing worldwide for indications including breast cancer, lung cancer and gastrointestinal cancers. From the perspective of clinical effects, when ADC drugs are used to treat solid tumours, the response rate for solid tumour types is relatively low. For example, Trodelvy^{®} is an ADC drug targeting Trop-2, and in the treatment of triple-negative breast cancer (TNBC), the objective response rate (ORR) is 27% to 35%; Tivdak^{®} is an ADC drug targeting TF, and in the treatment of recurrent or malignant cervical cancer, the ORR is 24%. At the same time, since ADC is conjugated with cytotoxic compound payloads, toxicities are observed in clinical use, such as haematological adverse reactions, including pancytopenia, neutropenia, thrombocytopenia, etc., as well as peripheral neuropathy, hepatotoxicity, pulmonary toxicity, cardiotoxicity, etc., thereby limiting clinical dosing and resulting in suboptimal therapeutic effects in certain indications. Accordingly, additional treatment modalities for ADC drugs, for example use in combination with drugs having different mechanisms of action, remain to be explored in order to further improve therapeutic effects.

Immune checkpoints, as immunosuppressive pathways, are crucial for maintaining self-tolerance and regulating the duration and extent of immune responses in peripheral tissues. However, these pathways can be "hijacked" by tumours and continuously activated, suppressing anti-tumour immunity and promoting tumour development (Pardoll DM. The blockade of immune checkpoints in cancer immunotherapy. Nat Rev Cancer. 2012;12(4):252-264); Haanen JB, Robert C. Immune Checkpoint Inhibitors. Prog Tumor Res. 2015;42:55-66). Programmed death-1 (PD-1) and its ligand (PD-L1) are the most widely applied immune checkpoint inhibitor (ICI) targets at present and are approved for the clinical treatment of multiple tumours, but due to the heterogeneity of tumours and the complexity of the tumour microenvironment, the overall efficacy of immune checkpoint inhibitor treatment is relatively low (Taube JM, Klein A, Brahmer JR, et al. Association of PD-1, PD-1 ligands, and other features of the tumour immune microenvironment with response to anti-PD-1 therapy. Clin Cancer Res. 2014;20(19):5064-5074; Restifo NP, Smyth MJ, Snyder A. Acquired resistance to immunotherapy and future challenges. Nat Rev Cancer. 2016;16(2):121-126), and for most cancer types, only 20% to 30% of patients exhibit an immune response.

Small-molecule cytotoxic drugs have been used clinically for many years and are first-line treatment methods for a variety of diseases. Although such drugs may show significant efficacy at an early stage of treatment, they are highly toxic, poorly tolerated during long-term dosing, and prone to inducing drug resistance.

### SUMMARY OF THE INVENTION

In view of the shortcomings of immune checkpoint inhibitors and small-molecule cytotoxic agents in the treatment of cancer, the present disclosure provides a combination of an antibody-drug conjugate and additional therapeutic agent(s), with a view to achieving excellent anti-tumour effects in the treatment of cancer in an individual, such as a human or animal, for example enhanced efficacy, increased durability of therapeutic response and/or reduced dose-dependent toxicity.

In a first aspect, the present disclosure provides a method of treating cancer, the method comprising administering to a subject, for example to a subject in need thereof, an effective amount of:
(a) an anti-B7H3 antibody-drug conjugate, which comprises an anti-B7H3 antibody or antigen-binding fragment thereof and a camptothecin drug, wherein the anti-B7H3 antibody or antigen-binding fragment thereof comprises a heavy chain variable region and a light chain variable region, wherein:
   the heavy chain variable region comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme;
   and (b) an additional therapeutic agent, wherein the additional therapeutic agent is selected from an anti-PD-1 antibody or antigen-binding fragment thereof, a chemotherapeutic drug, or one or both of any of the foregoing.

In some embodiments, the anti-B7H3 antibody or antigen-binding fragment thereof of the anti-B7H3 antibody-drug conjugate comprises a heavy chain variable region and a light chain variable region, wherein the heavy chain variable region comprises an amino acid sequence having at least 85% sequence identity to the amino acid sequence set forth in SEQ ID NO: 7, and the light chain variable region comprises an amino acid sequence having at least 85% sequence identity to the amino acid sequence set forth in SEQ ID NO: 8.

In some embodiments, the amino acid sequence of the heavy chain variable region is an amino acid sequence having at least 85%, at least 90%, at least 95%, at least 96%, at least 97%, at least 98%, at least 99%, at least 99.2%, at least 99.5%, at least 99.7%, at least 99.9% sequence identity with SEQ ID NO: 7, and the amino acid sequence of the light chain variable region is an amino acid sequence having at least 85%, at least 90%, at least 95%, at least 96%, at least 97%, at least 98%, at least 99%, at least 99.2%, at least 99.5%, at least 99.7%, at least 99.9% sequence identity with SEQ ID NO: 8.

In some embodiments, the heavy chain variable region of the anti-B7H3 antibody or antigen-binding fragment thereof comprises an amino acid sequence set forth in SEQ ID NO: 7, and the light chain variable region comprises an amino acid sequence set forth in SEQ ID NO: 8.

In some embodiments, the amino acid sequence of the heavy chain variable region of the anti-B7H3 antibody or antigen-binding fragment thereof is set forth in SEQ ID NO: 7, and the amino acid sequence of the light chain variable region is set forth in SEQ ID NO: 8.

In some embodiments, the anti-B7H3 antibody comprises a heavy chain set forth in SEQ ID NO: 9, and a light chain set forth in SEQ ID NO: 10.

In some embodiments, the anti-B7H3 antibody comprises a heavy chain having an amino acid sequence having at least 85%, at least 90%, at least 95%, at least 96%, at least 97%, at least 98%, at least 99%, at least 99.2%, at least 99.5%, at least 99.7%, at least 99.9% sequence identity with SEQ ID NO: 9, and a light chain having an amino acid sequence having at least 85%, at least 90%, at least 95%, at least 96%, at least 97%, at least 98%, at least 99%, at least 99.2%, at least 99.5%, at least 99.7%, at least 99.9% sequence identity with SEQ ID NO: 10.

In some embodiments, the anti-B7H3 antibody or antigen-binding fragment thereof is the antibody designated 2E3-02 in WO2022170971.

In a second aspect, the present disclosure provides a method of treating cancer, the method comprising administering to a subject, for example to a subject in need thereof, an effective amount of:
a) an anti-B7H3 antibody-drug conjugate, wherein the anti-B7H3 antibody-drug conjugate is an antibody-drug conjugate having the following formula, a pharmaceutically acceptable salt, stereoisomer or metabolite thereof, or a solvate of any of the foregoing:

   Tb-(L-D)q,

   wherein:
   Tb is an anti-B7H3 antibody or antigen-binding fragment thereof; preferably, Tb is the anti-B7H3 antibody or antigen-binding fragment thereof according to the first aspect described above; L is a linker and has the structure as the following formula: 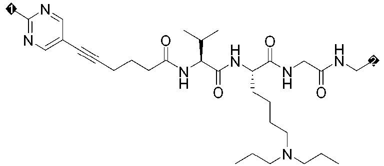
   position 1 is linked to Tb, and position 2 is linked to D;
   D is a bioactive molecular fragment, for example a camptothecin drug;
   q is selected from any numerical value ranging from 0.1 to 16.0; preferably, q is selected from 1, 2, 3, 4, 5, 6, 7, 8, 9 and 10; more preferably, q is 2, 4, 6 or 8;
   and (b) an additional therapeutic agent, wherein the additional therapeutic agent is selected from an anti-PD-1 antibody or antigen-binding fragment thereof, a chemotherapeutic drug, or one or both of any of the foregoing.

In some embodiments, the anti-B7H3 antibody-drug conjugate is an antibody-drug conjugate having the structure shown in the following formula I, a pharmaceutically acceptable salt, stereoisomer or metabolite thereof, or a solvate of any of the foregoing: 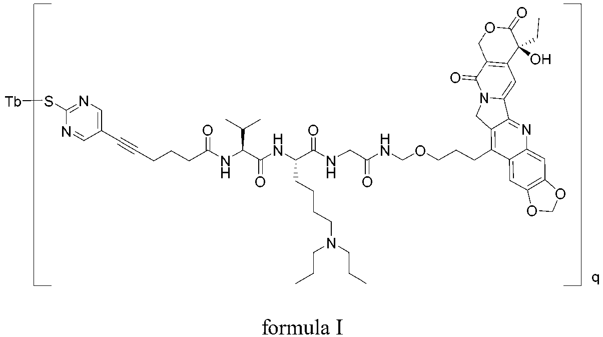 wherein:
S is a sulphur atom on Tb;
Tb is an anti-B7H3 antibody or antigen-binding fragment thereof;
q is selected from any numerical value ranging from 0.1 to 16.0; preferably, q is selected from 1, 2, 3, 4, 5, 6, 7, 8, 9, 10; more preferably, q is 2, 4, 6 or 8.

In some other embodiments, the antibody-drug conjugate may further be selected from: 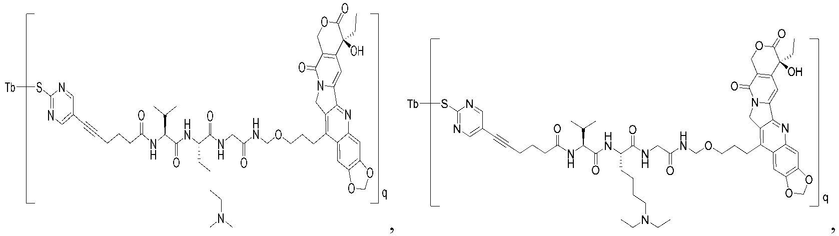 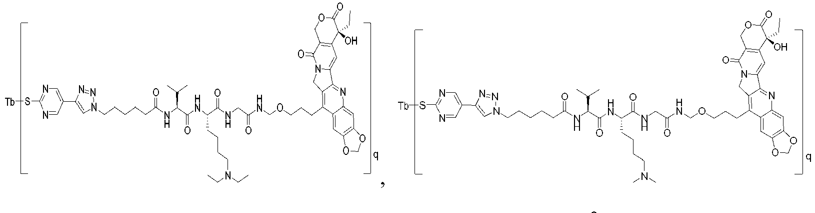 and 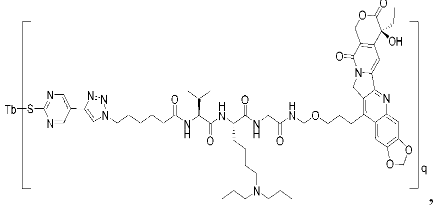
wherein Tb is an anti-B7H3 antibody or antigen-binding fragment thereof;
S is a sulphur atom on Tb;
q is a drug-antibody coupling ratio, and is selected from any numerical value ranging from 0.1 to 16.0, preferably, 1 to 10; preferably, q is 1, 2, 3, 4, 5, 6, 7 or 8; more preferably, q is 2, 4, 6 or 8.

In some embodiments, the anti-B7H3 antibody-drug conjugate is an antibody-drug conjugate having the structure shown in the following formula II, a pharmaceutically acceptable salt, stereoisomer or metabolite thereof, or a solvate of any of the foregoing, 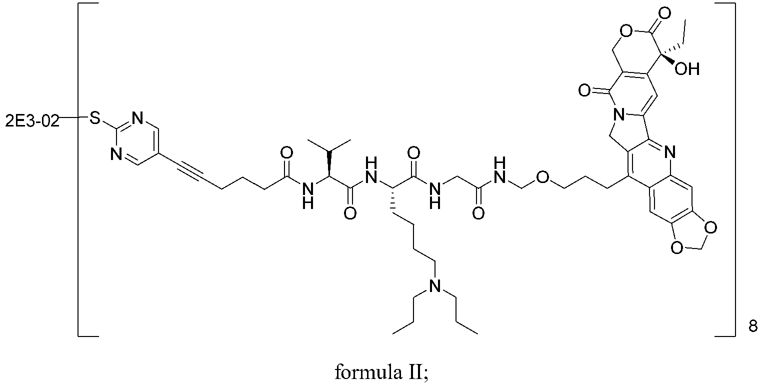 wherein S is a sulphur atom on Tb.

In some embodiments, the anti-B7H3 antibody or antigen-binding fragment thereof as well as the monoclonal antibody or antigen-binding fragment thereof comprise: Fab, Fab', F(ab')₂, Fd, Fv (for example, scFv), dAb, a complementarity-determining region fragment, a non-human antibody, a humanised antibody, a chimeric antibody, a fully human antibody, a probody, a monoclonal antibody, a bispecific antibody or a multispecific antibody.

In some embodiments, Tb is an antibody having B7H3-2Ig and/or B7H3-4Ig or an antigen-binding fragment thereof.

In some embodiments, Tb is an antibody having B7H3-4Ig binding activity higher than B7H3-2Ig binding activity or an antigen-binding fragment thereof.

In some embodiments, Tb is a non-human antibody, a humanised antibody, a chimeric antibody or a fully human antibody.

In some embodiments, Tb is a monoclonal antibody, a bispecific antibody or a multispecific antibody.

In some embodiments, Tb is a monoclonal antibody or antigen-binding fragment thereof.

In some embodiments, the anti-B7H3 antibody or antigen-binding fragment thereof is the antibodies designated 1D1, 1D1-01, 2E3, 2E3-02 in WO2022170971, enoblituzumab, mirzotamab, omburtamab, the antibody designated M30-H1-L4 in CN 103687945B, the antibody designated mAb-C-DUBA in CN 109069633A, and the anti-B7H3 antibody or antigen-binding fragment thereof with reference to CN112521512, WO2021027674, WO2021021543, WO2021006619, CN111662384, CN111454357, WO2020151384, WO2020140094, WO2020103100, WO2020102779, WO2020063673, WO2020047257, WO2020041626, CN110684790, CN110642948, WO2019225787, WO2019226017, US20190338030, CN110305213, WO2018209346, WO2018177393, US9150656, WO2016106004, WO2016044383, WO2016033225, WO2015181267, US20120294796, WO2011109400, CN101104639, WO2004093894, WO2002010187 or WO2001018021.

In some embodiments, the anti-B7H3 antibody or antigen-binding fragment thereof is the antibody designated 2E3-02 in WO2022170971.

In some embodiments, the anti-B7H3 antibody is the 2E3-02 antibody, and the 2E3-02 antibody comprises a heavy chain set forth in SEQ ID NO: 9 and a light chain set forth in SEQ ID NO: 10.

In some embodiments, the antibody or antigen-binding fragment further comprises a framework region derived from human or murine immunoglobulin.

In some embodiments, the additional therapeutic agent is an anti-PD-1 antibody or antigen-binding fragment thereof.

In some embodiments, the additional therapeutic agent is an anti-PD-1 antibody or antigen-binding fragment thereof and a chemotherapeutic drug.

In some embodiments, the additional therapeutic agent satisfies the following one or more conditions:
i. the anti-PD-1 antibody or antigen-binding fragment thereof is selected from AK103 (HX008), treprinumab, sintilimab, camrelizumab, tislelizumab, nivolumab (Opdivo), pembrolizumab (Keytruda), nofazinlimab, serplulimab (HLX10 or HANSIZHUANG), geptanolimab, lipustobart, BAT-1306, finotonlimab, rulonilimab, SG001, zimberelimab, spartalizumab, cemiplimab, STI-A1110, or one or more of any of the foregoing; the anti-PD-1 antibody may also be the Bio X Cell BE0146 antibody;
ii. the chemotherapeutic drug is selected from platinum-based drugs; preferably, the platinum-based chemotherapeutic drugs are selected from cisplatin, carboplatin, sulfatodiaminocyclohexane platin, nedaplatin, oxaliplatin, lobaplatin, satraplatin, miboplatin, enloplatin, iproplatin, dicycloplatin, or a combination of any of the foregoing;
iii. the anti-PD-1 antibody is not serplulimab;
iv. the anti-PD-1 antibody is not pembrolizumab (Keytruda);
v. the anti-PD-1 antibody is not treprinumab.

In some preferred embodiments, the anti-PD-1 antibody or antigen-binding fragment thereof is selected from pembrolizumab (Keytruda), serplulimab (HLX10 or HANSIZHUANG), treprinumab, or the Bio X Cell BE0146 antibody.

In some preferred embodiments, the anti-PD-1 antibody or antigen-binding fragment thereof is selected from pembrolizumab (Keytruda) and serplulimab (HLX10 or HANSIZHUANG).

In some embodiments, when the anti-PD-1 antibody is serplulimab, the anti-B7H3 antibody-drug conjugate is not the antibody-drug conjugate having the structure shown in formula I, a pharmaceutically acceptable salt thereof: 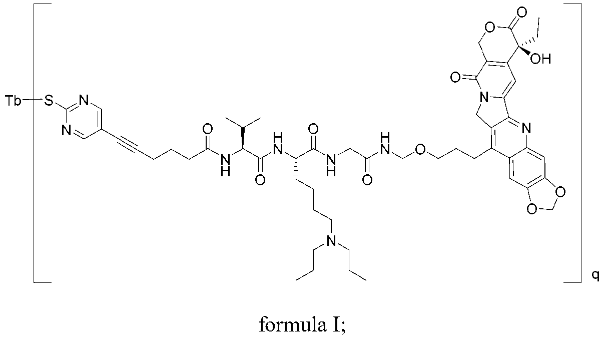
wherein S is a sulphur atom on Tb, Tb is an anti-B7H3 antibody or antigen-binding fragment thereof, and q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence of SEQ ID NO: 1;
      (ii) CDR-H2 comprising the amino acid sequence of SEQ ID NO: 2; and
      (iii) CDR-H3 comprising the amino acid sequence of SEQ ID NO: 3; and
   the light chain variable region (VL) comprises:
      (i) CDR-L1 comprising the amino acid sequence of SEQ ID NO: 4;
      (ii) CDR-L2 comprising the amino acid sequence of SEQ ID NO: 5; and
      (iii) CDR-L3 comprising the amino acid sequence of SEQ ID NO: 6;
wherein the CDRs are determined in accordance with the Kabat definition scheme.

In some embodiments, the method comprises administering to the subject an anti-B7H3 antibody-drug conjugate and an anti-PD-1 antibody or antigen-binding fragment thereof.

In some embodiments, the method comprises administering to the subject an anti-B7H3 antibody-drug conjugate, an anti-PD-1 antibody or antigen-binding fragment thereof, and a platinum-based drug.

In some embodiments, the platinum-based drug is selected from carboplatin.

In some embodiments, the platinum-based drug is selected from cisplatin.

In some embodiments, the method of treating cancer comprises administering to the subject:
(a) an anti-B7H3 antibody-drug conjugate, wherein the anti-B7H3 antibody-drug conjugate is an antibody-drug conjugate having the structure shown in the following formula I, a pharmaceutically acceptable salt, stereoisomer or metabolite thereof, or a solvate of any of the foregoing: 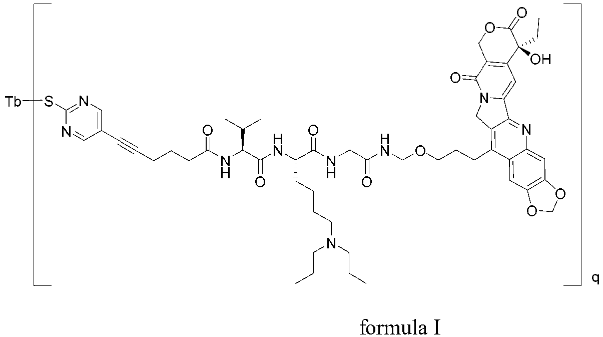
   wherein: S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
   the anti-B7H3 antibody or antigen-binding fragment thereof comprises a heavy chain variable region and a light chain variable region, wherein:
      the heavy chain variable region (VH) comprises:
         (i) CDR-H1 comprising the amino acid sequence set forth in SEQ ID NO: 1;
         (ii) CDR-H2 comprising the amino acid sequence set forth in SEQ ID NO: 2; and
         (iii) CDR-H3 comprising the amino acid sequence set forth in SEQ ID NO: 3; and
      the light chain variable region (VL) comprises:
         (i) CDR-L1 comprising the amino acid sequence set forth in SEQ ID NO: 4;
         (ii) CDR-L2 comprising the amino acid sequence set forth in SEQ ID NO: 5; and
         (iii) CDR-L3 comprising the amino acid sequence set forth in SEQ ID NO: 6;
   wherein the CDRs are determined in accordance with the Kabat definition scheme;
   and (b) an anti-PD-1 antibody or antigen-binding fragment thereof, wherein the anti-PD-1 antibody is serplulimab (HLX10 or HANSIZHUANG).

In some embodiments, the method of treating cancer further comprises administering to the subject: (c) a platinum-based drug. In some embodiments, the platinum-based drug is selected from carboplatin. In some embodiments, the platinum-based drug is selected from cisplatin.

In some embodiments, the method of treating cancer comprises administering to the subject:
(a) an anti-B7H3 antibody-drug conjugate, wherein the anti-B7H3 antibody-drug conjugate is an antibody-drug conjugate having the structure shown in the following formula I, a pharmaceutically acceptable salt, stereoisomer or metabolite thereof, or a solvate of any of the foregoing: 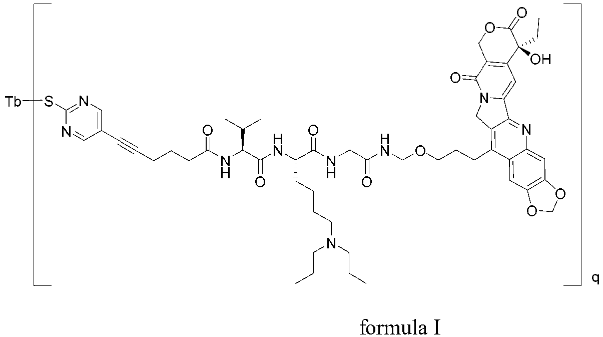
   wherein: S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
   the anti-B7H3 antibody or antigen-binding fragment thereof comprises a heavy chain variable region and a light chain variable region, wherein:
      the heavy chain variable region (VH) comprises:
         (i) CDR-H1 comprising the amino acid sequence set forth in SEQ ID NO: 1;
         (ii) CDR-H2 comprising the amino acid sequence set forth in SEQ ID NO: 2; and
         (iii) CDR-H3 comprising the amino acid sequence set forth in SEQ ID NO: 3; and
      the light chain variable region (VL) comprises:
         (i) CDR-L1 comprising the amino acid sequence set forth in SEQ ID NO: 4;
         (ii) CDR-L2 comprising the amino acid sequence set forth in SEQ ID NO: 5; and
         (iii) CDR-L3 comprising the amino acid sequence set forth in SEQ ID NO: 6;
   wherein the CDRs are determined in accordance with the Kabat definition scheme;
   and (b) an anti-PD-1 antibody or antigen-binding fragment thereof, wherein the anti-PD-1 antibody is pembrolizumab (Keytruda).
   In some embodiments, the method of treating cancer further comprises administering to the subject: (c) a platinum-based drug. In some embodiments, the platinum-based drug is selected from carboplatin. In some embodiments, the platinum-based drug is selected from cisplatin.

In some embodiments, the anti-B7H3 antibody-drug conjugate, the pharmaceutically acceptable salt, stereoisomer or metabolite thereof, or the solvate of any of the foregoing is administered at a dose of 0.1-15 mg/kg individual body weight.

In some embodiments, the anti-B7H3 antibody-drug conjugate is administered to the subject at a dose within the following ranges: 0.1 to 10 mg/kg, 0.2 to 8 mg/kg, 0.3 to 6 mg/kg, 0.4 to 4 mg/kg, or 0.5 to 3 mg/kg;
in some embodiments, the anti-B7H3 antibody-drug conjugate is administered to the subject at a dose as follows: 0.4 mg/kg, 0.5 mg/kg, 0.6 mg/kg, 0.8 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 4.0 mg/kg, 5.0 mg/kg.

In some embodiments, the anti-B7H3 antibody-drug conjugate is administered to the subject at a dose of about 1.2 mg/kg.

In some embodiments, the anti-B7H3 antibody-drug conjugate is administered to the subject at a dose of about 1.4 mg/kg.

In some embodiments, the anti-B7H3 antibody-drug conjugate is administered to the subject at a dose of about 1.6 mg/kg.

In some embodiments, the anti-B7H3 antibody-drug conjugate is administered to the subject at a dose of about 1.8 mg/kg.

In some embodiments, the anti-B7H3 antibody-drug conjugate is administered to the subject at a dose of about 2.0 mg/kg.

In some embodiments, the anti-B7H3 antibody-drug conjugate is administered to the subject at a dose of about 2.2 mg/kg.

In some embodiments, the anti-B7H3 antibody-drug conjugate is administered to the subject at a dose of about 2.4 mg/kg.

In some embodiments, the anti-B7H3 antibody-drug conjugate is administered to the subject at a dose of about 2.6 mg/kg.

In some embodiments, the anti-B7H3 antibody-drug conjugate is administered to the subject at a dose of about 2.8 mg/kg.

In some embodiments, the anti-B7H3 antibody-drug conjugate is administered to the subject at a dose of about 3.0 mg/kg.

In some embodiments, wherein the anti-PD-1 antibody or antigen-binding fragment thereof is administered to the subject at a flat dose in the range of about 50 mg to about 650 mg or at a dose of 1 to 10 mg/kg.

In some embodiments, wherein the anti-PD-1 antibody or antigen-binding fragment thereof is administered to the subject at a flat dose in the following ranges: 100 to 600 mg, 100 to 500 mg, 100 to 400 mg, 200 to 600 mg, 200 to 500 mg, 200 to 400 mg, 150 to 450 mg, 150 to 250 mg, 250 to 350 mg, 350 to 450 mg, 450 to 550 mg, 550 to 650 mg.

In some embodiments, wherein the anti-PD-1 antibody or antigen-binding fragment thereof is administered to the subject at a flat dose in the following ranges: 100 mg, 200 mg, 300 mg, 400 mg, 500 mg, 600 mg.

In some embodiments, wherein the anti-PD-1 antibody or antigen-binding fragment thereof is administered to the subject at a flat dose of about 100 mg.

In some embodiments, wherein the anti-PD-1 antibody or antigen-binding fragment thereof is administered to the subject at a flat dose of about 200 mg.

In some embodiments, wherein the anti-PD-1 antibody or antigen-binding fragment thereof is administered to the subject at a flat dose of about 400 mg.

In some embodiments, wherein the anti-PD-1 antibody or antigen-binding fragment thereof is administered to the subject at a flat dose of about 600 mg.

In some embodiments, wherein the anti-PD-1 antibody or antigen-binding fragment thereof is administered to the subject at the following dose: 1.0 mg/kg, 1.2 mg/kg, 1.4 mg/kg, 1.6 mg/kg, 1.8 mg/kg, 2.0 mg/kg, 2.4 mg/kg, 2.8 mg/kg, 3.0 mg/kg, 3.5 mg/kg, 4.0 mg/kg, 4.5 mg/kg, 5.0 mg/kg.

In some embodiments, wherein the anti-PD-1 antibody or antigen-binding fragment thereof is administered to the subject at a dose of about 2.0 mg/kg.

In some embodiments, wherein the anti-PD-1 antibody or antigen-binding fragment thereof is administered to the subject at a dose of about 4.0 mg/kg.

In some embodiments, wherein the anti-PD-1 antibody or antigen-binding fragment thereof is administered to the subject at a dose of about 4.5 mg/kg.

In some embodiments, wherein the anti-PD-1 antibody or antigen-binding fragment thereof is administered to the subject at a dose of about 6.0 mg/kg.

In some embodiments, wherein the platinum-based drug is administered:
i. to the subject at a dose in the range of about 50 to 150 mg/m²;
   preferably, the platinum-based drug is administered to the subject at the following dose: 50 mg/m², 70 mg/m², 75 mg/m², 80 mg/m², 100 mg/m², 120 mg/m² or 150 mg/m²;
   or
ii. at a dose per administration, calculated as an area under the curve (AUC), selected from 1 to 10 mg/ml/min;
preferably, the platinum-based drug is administered to the subject at the following dose (i.e. injection rate): 3 mg/mL/min, 4 mg/mL/min, 5 mg/mL/min, 6 mg/mL/min or 7 mg/mL/min.

In some embodiments, the platinum-based drug is administered to the subject at the following dose: 50 mg/m², 70 mg/m², 75 mg/m², 80 mg/m², 100 mg/m², 120 mg/m² or 150 mg/m².

In some embodiments, the platinum-based drug is administered to the subject at a dose of about 70 mg/m².

In some embodiments, the platinum-based drug is administered to the subject at a dose of about 75 mg/m².

In some embodiments, the platinum-based drug is administered to the subject at a dose of about 80 mg/m².

In some embodiments, the dose of the carboplatin per administration, calculated as an area under the curve (AUC), is selected from 1 to 10 mg/ml/min, preferably from 1 mg/ml/min, 2 mg/ml/min, 2.5 mg/ml/min, 3 mg/ml/min, 3.75 mg/ml/min, 4 mg/ml/min, 5 mg/ml/min, 6 mg/ml/min, 7 mg/ml/min, 8 mg/ml/min. In some embodiments, the dose of the carboplatin per administration is 3 mg/ml/min, 4 mg/ml/min, 5 mg/ml/min, 6 mg/ml/min, or 7 mg/ml/min.

In some embodiments, the dose of the cisplatin per administration is selected from 50 to 150 mg/m², preferably from 50 mg/m², 70 mg/m², 75 mg/m², 80 mg/m², 100 mg/m², 120 mg/m² and 150 mg/m².

In some embodiments, wherein the anti-B7H3 antibody-drug conjugate, the anti-PD-1 antibody or antigen-binding fragment thereof, and the platinum-based drug is administered in a dosing cycle of 7 to 42 days. Preferably, the dosing cycle is 21 to 42 days.

In some embodiments, wherein the anti-B7H3 antibody-drug conjugate, the anti-PD-1 antibody or antigen-binding fragment thereof, and the platinum-based drug is administered in a dosing cycle of 14 days, 21 days, 28 days, 35 days, or 42 days.

In some embodiments, the anti-B7H3 antibody-drug conjugate is administered at a dosing frequency of once daily, once weekly, once every two weeks, once every three weeks, twice every three weeks, once every four weeks, once monthly, once every five weeks, or once every six weeks.

In some embodiments, the anti-B7H3 antibody-drug conjugate is administered at a dosing cycle of one week, two weeks, three weeks, one month, two months, three months, four months, five months, six months, or longer; optionally, the duration of each dosing cycle is the same or different, and the interval between each dosing cycle is the same or different.

In some embodiments, the PD-1 antibody or antigen-binding fragment thereof is administered at a dosing frequency of once daily, once weekly, once every two weeks, once every three weeks, once every four weeks or once monthly, once every five weeks, once every six weeks.

In some embodiments, the PD-1 antibody or antigen-binding fragment thereof is administered at a dosing cycle of one week, two weeks, three weeks, one month, two months, three months, four months, five months, six months, or longer; optionally, the duration of each dosing cycle is the same or different, and the interval between each dosing cycle is the same or different.

In some embodiments, the platinum-based drug (for example, carboplatin or cisplatin) is administered at a dosing frequency of once daily, once weekly, once every two weeks, once every three weeks, once every four weeks or once monthly, once every five weeks, once every six weeks.

In some embodiments, the platinum-based drug (for example, carboplatin or cisplatin) is administered at a dosing cycle of one week, two weeks, three weeks, one month, two months, three months, four months, five months, six months, or longer; optionally, the duration of each dosing cycle is the same or different, and the interval between each dosing cycle is the same or different.

In some embodiments, the anti-B7H3 antibody-drug conjugate and the additional therapeutic agent are administered simultaneously.

In some embodiments, the anti-B7H3 antibody-drug conjugate and the additional therapeutic agent are administered separately.

In some embodiments, the anti-B7H3 antibody-drug conjugate and the additional therapeutic agent are administered sequentially.

In some embodiments, the anti-B7H3 antibody-drug conjugate is administered first, followed by the administration of the additional therapeutic agent.

In some embodiments, the cancer is selected from a solid tumour.

In some embodiments, the cancer is selected from nasopharyngeal carcinoma, colorectal cancer, non-small cell lung cancer, small cell lung cancer, oesophageal squamous cell carcinoma, prostate cancer, head and neck squamous cell carcinoma.

In some embodiments, the cancer is selected from nasopharyngeal carcinoma, non-small cell lung cancer, small cell lung cancer.

In some embodiments, the cancer is selected from non-small cell lung cancer.

In some embodiments, the non-small cell lung cancer (NSCLC) is selected from squamous NSCLC, adenocarcinoma NSCLC, pulmonary lymphoepithelioma-like carcinoma, large cell lung cancer.

In some embodiments, the non-small cell lung cancer (NSCLC) is selected from those positive for driver genes or negative for driver genes.

In some embodiments, the nasopharyngeal carcinoma is selected from keratinising squamous cell carcinoma of the nasopharynx, non-keratinising nasopharyngeal carcinoma (including differentiated or undifferentiated types), basaloid squamous cell carcinoma of the nasopharynx, nasopharyngeal carcinoma in situ, invasive nasopharyngeal carcinoma (including, but not limited to, squamous cell carcinoma, adenocarcinoma, microinvasive carcinoma, vesicular nucleus carcinoma or undifferentiated nasopharyngeal carcinoma).

In some embodiments, the non-keratinising nasopharyngeal carcinoma includes differentiated or undifferentiated types.

In some embodiments, the invasive nasopharyngeal carcinoma includes, but is not limited to, squamous cell carcinoma, adenocarcinoma, microinvasive carcinoma, vesicular nucleus carcinoma or undifferentiated nasopharyngeal carcinoma.

In some embodiments, the subject is a patient with locally recurrent or metastatic nasopharyngeal carcinoma who has previously failed treatment with a platinum-containing regimen.

In some embodiments, the subject is a patient with locally recurrent or metastatic nasopharyngeal carcinoma who has not received systemic treatment.

In some embodiments, the subject is a patient with small cell lung cancer who has not received systemic treatment.

In some embodiments, the subject is a patient with locally advanced or metastatic non-small cell lung cancer who has previously failed treatment with a platinum-containing regimen and who is negative for epidermal growth factor receptor (EGFR) sensitising mutations, negative for anaplastic lymphoma kinase (ALK), and/or negative for c-ros proto-oncogene (ROS1).

In some embodiments, the subject is a patient with locally advanced or metastatic non-small cell lung cancer who has previously failed treatment with a platinum-containing regimen and who is negative for epidermal growth factor receptor (EGFR) sensitising mutations and negative for anaplastic lymphoma kinase (ALK)/c-ros proto-oncogene (ROS1).

In some embodiments, the subject is a patient with locally advanced or metastatic non-small cell lung cancer who has not received systemic treatment and who is negative for EGFR sensitising mutations, negative for ALK and/or negative for c-ros proto-oncogene (ROS1).

In some embodiments, the subject is a patient with locally advanced or metastatic non-small cell lung cancer who has not received systemic treatment and who is negative for EGFR sensitising mutations and negative for ALK/c-ros proto-oncogene (ROS1).

In some embodiments, the anti-B7H3 antibody-drug conjugate and the additional therapeutic agent are present in the form of unit dosage.

In some embodiments, the anti-B7H3 antibody-drug conjugate and the additional therapeutic agent are present in the same dosage unit.

In some embodiments, the anti-B7H3 antibody-drug conjugate and the additional therapeutic agent are present in different dosage units.

In some embodiments, the route of dosing may be oral dosing, parenteral dosing, transdermal dosing, and the parenteral dosing includes, but is not limited to, intravenous injection, subcutaneous injection, intramuscular injection.

In some embodiments, the route of administration of the anti-B7H3 antibody-drug conjugate and the additional therapeutic agent is intravenous injection.

In some embodiments, the anti-B7H3 antibody-drug conjugate, or a pharmaceutically acceptable salt, stereoisomer or metabolite thereof or a solvate of any of the foregoing is administered in a manner of injection; and prior to injection, the anti-B7H3 antibody-drug conjugate, or a pharmaceutically acceptable salt, stereoisomer or metabolite thereof or a solvate of any of the foregoing needs to be formulated into an injectable form, the preferred injection form is an injection solution or lyophilised powder, comprising the anti-B7H3 antibody-drug conjugate, a pharmaceutically acceptable salt, stereoisomer or metabolite thereof, or a solvate of any of the foregoing, and optionally, a buffering agent, a stabiliser, a pH adjusting agent and a surfactant, wherein the buffering agent may be selected from one or more of acetate, citrate, succinate and phosphate; the stabiliser may be selected from a sugar or an amino acid, preferably a disaccharide, such as sucrose, lactose, trehalose or maltose; the pH adjusting agent may be selected from one or more of sodium hydroxide, lithium hydroxide and potassium hydroxide; the surfactant may be selected from polyoxyethylene hydrogenated castor oil, glycerol fatty acid ester and polyoxyethylene sorbitan fatty acid ester, and preferably the polyoxyethylene sorbitan fatty acid ester is polysorbate 20, 40, 60 or 80, most preferably polysorbate 20.

In some embodiments, wherein one or more therapeutic effects in the subject are improved relative to the baseline following administration of the anti-B7H3 antibody-drug conjugate and the anti-PD-1 antibody or antigen-binding fragment thereof, wherein the one or more therapeutic effects are selected from size of tumour derived from the cancer, objective response rate (ORR), depth of response (DpR), disease control rate (DCR), duration of response (DoR), time to response (TTR), progression-free survival (PFS), and overall survival (OS).

In some embodiments, the size of tumour derived from the cancer of the subject is reduced by at least about 10%, at least about 15%, at least about 20%, at least about 25%, at least about 30%, at least about 35%, at least about 40%, at least about 45%, at least about 50%, at least about 60%, at least about 70%, or at least about 80%, relative to the size of tumour prior to administration of the anti-B7H3 antibody-drug conjugate and the anti-PD-1 antibody or antigen-binding fragment thereof.

In a third aspect, the present disclosure further provides a pharmaceutical composition, comprising an anti-B7H3 antibody-drug conjugate and an additional therapeutic agent, wherein the additional therapeutic agent is selected from one or both of an anti-PD-1 antibody or antigen-binding fragment thereof and a chemotherapeutic drug, and the anti-B7H3 antibody-drug conjugate, the anti-PD-1 antibody or antigen-binding fragment thereof, and the chemotherapeutic drug are as defined herein.

In some embodiments, the pharmaceutical composition is a pharmaceutical composition for treating cancer, and the cancer is as defined above.

In some embodiments, the pharmaceutical composition further comprises one or more pharmaceutically acceptable excipients, diluents, or carriers.

In some embodiments, the pharmaceutical composition comprises an anti-B7H3 antibody-drug conjugate and an additional therapeutic agent, wherein the additional therapeutic agent is selected from: i. serplulimab, or ii. serplulimab and a platinum-based drug;
the anti-B7H3 antibody-drug conjugate is an antibody-drug conjugate having the structure shown in formula I, a pharmaceutically acceptable salt thereof: 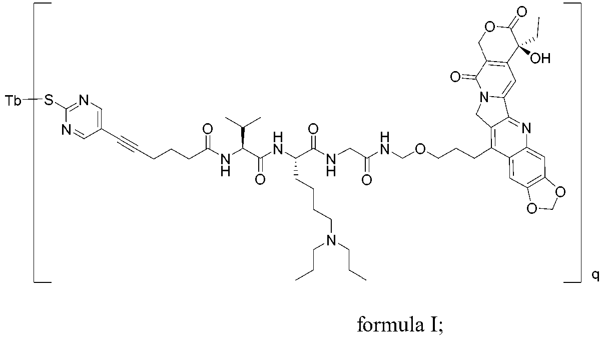
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, the pharmaceutical composition comprises an anti-B7H3 antibody-drug conjugate and an additional therapeutic agent, wherein the additional therapeutic agent is selected from: i. pembrolizumab (Keytruda); or ii. pembrolizumab (Keytruda) and a platinum-based drug;
the anti-B7H3 antibody-drug conjugate is an antibody-drug conjugate having the structure shown in formula I, a pharmaceutically acceptable salt thereof: 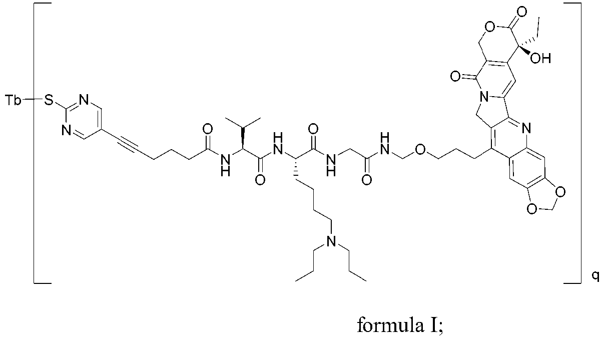
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In a fourth aspect, the present disclosure further provides a kit, comprising an anti-B7H3 antibody-drug conjugate and an additional therapeutic agent, wherein the additional therapeutic agent is selected from one or both of an anti-PD-1 antibody or antigen-binding fragment thereof and a chemotherapeutic drug, and the anti-B7H3 antibody-drug conjugate, the anti-PD-1 antibody or antigen-binding fragment thereof, and the chemotherapeutic drug are as defined herein.

In some embodiments, the kit is a kit for treating cancer, and the cancer is as defined above.

In some embodiments, the kit comprises an anti-B7H3 antibody-drug conjugate and an additional therapeutic agent, wherein the additional therapeutic agent is selected from: i. serplulimab, or ii. serplulimab and a platinum-based drug;
the anti-B7H3 antibody-drug conjugate is an antibody-drug conjugate having the structure shown in formula I, a pharmaceutically acceptable salt thereof: 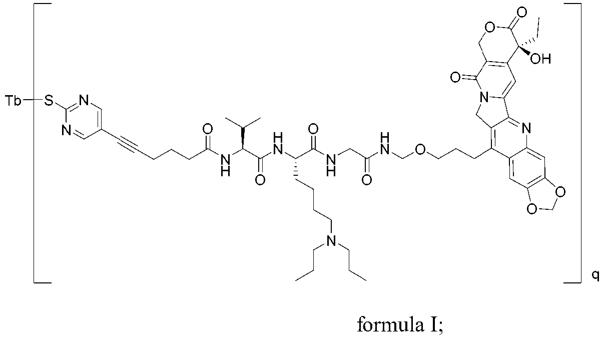
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, the kit comprises an anti-B7H3 antibody-drug conjugate and an additional therapeutic agent, wherein the additional therapeutic agent is selected from: i. pembrolizumab (Keytruda); or ii. pembrolizumab (Keytruda) and a platinum-based drug;
the anti-B7H3 antibody-drug conjugate is an antibody-drug conjugate having the structure shown in formula I, a pharmaceutically acceptable salt thereof: 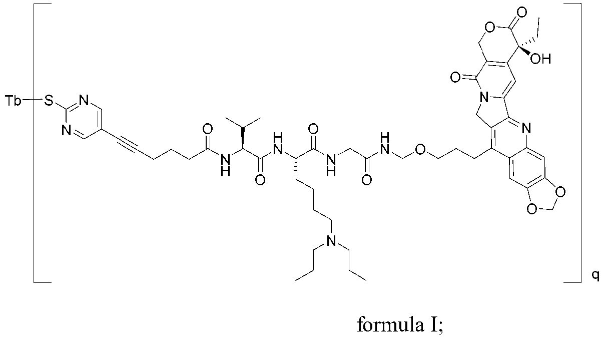
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, the kit comprises a container A and a container B, wherein the container A comprises an anti-B7H3 antibody-drug conjugate as an active ingredient, and the container B comprises the additional therapeutic agent as an active ingredient.

In a fifth aspect, the present disclosure further provides use of the kit according to the fourth aspect in the preparation of a drug for treating cancer.

In a sixth aspect, the present disclosure provides a pharmaceutical combination, comprising an anti-B7H3 antibody-drug conjugate and an additional therapeutic agent, wherein the additional therapeutic agent is selected from one or both of an anti-PD-1 antibody or antigen-binding fragment thereof and a chemotherapeutic drug, and the anti-B7H3 antibody-drug conjugate, the anti-PD-1 antibody or antigen-binding fragment thereof, and the chemotherapeutic drug are as defined above.

In a seventh aspect, the present disclosure provides use of a combination of an anti-B7H3 antibody-drug conjugate and an additional therapeutic agent in the preparation of a drug for treating cancer.

In some embodiments, there is further provided use of a combination of the anti-B7H3 antibody-drug conjugate and the anti-PD-1 antibody or antigen-binding fragment thereof as described above in the preparation of a drug for treating cancer.

In some embodiments, there is also provided use of a combination of the anti-B7H3 antibody-drug conjugate and one or both of the anti-PD-1 antibody or antigen-binding fragment thereof and a chemotherapeutic drug as described above in the preparation of a drug for treating cancer.

In one aspect, the present disclosure also provides the aforementioned kit or pharmaceutical combination for treating cancer.

In a further aspect, there is also provided use of an anti-B7H3 antibody-drug conjugate in the preparation of a drug for treating cancer, the anti-B7H3 antibody-drug conjugate is administered in combination with an additional therapeutic agent, and the anti-B7H3 antibody-drug conjugate is as defined above.

In a further aspect, there is also provided an anti-B7H3 antibody-drug conjugate for treating cancer, and the anti-B7H3 antibody-drug conjugate is as defined above and is administered in combination with an additional therapeutic agent.

In some embodiments, there is also provided an anti-B7H3 antibody-drug conjugate for use in combination with serplulimab (HLX10 or HANSIZHUANG) in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing; 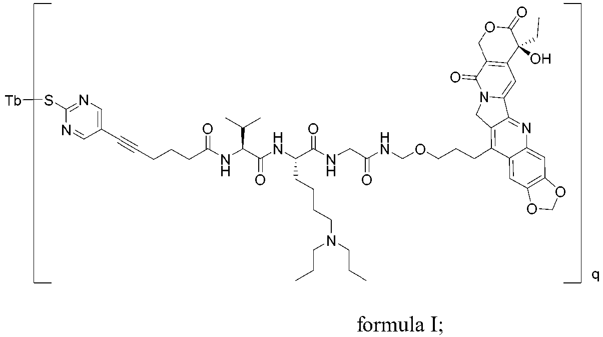
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided an anti-B7H3 antibody-drug conjugate for use in combination with serplulimab (HLX10 or HANSIZHUANG) and a platinum-based drug in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing: 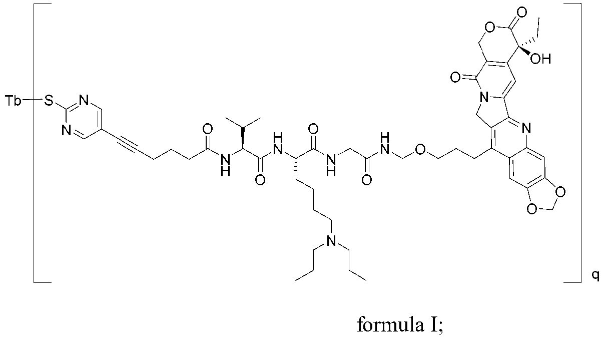
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided a PD-1 antibody for use in combination with an anti-B7H3 antibody-drug conjugate in treating cancer, wherein the PD-1 antibody is serplulimab, and the anti-B7H3 antibody-drug conjugate is an antibody-drug conjugate having the structure shown in formula I, a pharmaceutically acceptable salt, stereoisomer or metabolite thereof, or a solvate of any of the foregoing: 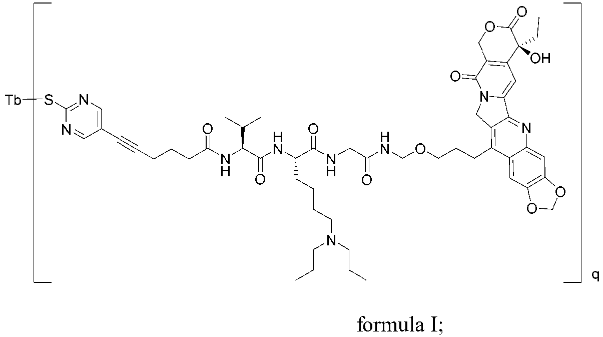
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided a PD-1 antibody for use in combination with an anti-B7H3 antibody-drug conjugate and a platinum-based drug in treating cancer, wherein the PD-1 antibody is serplulimab, and the anti-B7H3 antibody-drug conjugate is an antibody-drug conjugate having the structure shown in formula I, a pharmaceutically acceptable salt, stereoisomer or metabolite thereof, or a solvate of any of the foregoing: 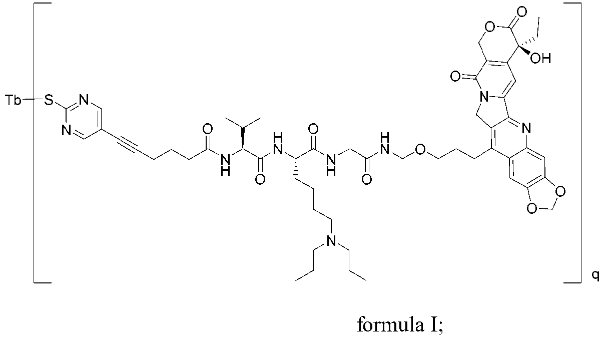
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided a combination of an anti-B7H3 antibody-drug conjugate and serplulimab (HLX10 or HANSIZHUANG) for use in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing: 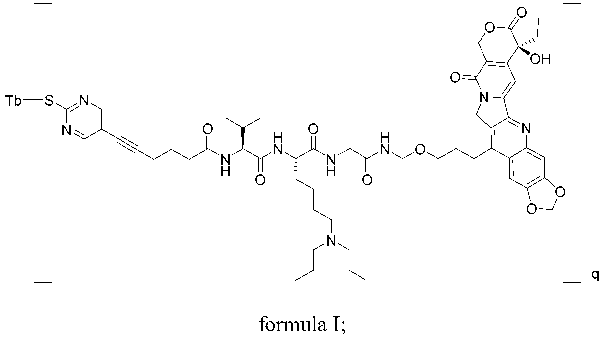
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided an anti-B7H3 antibody-drug conjugate for use in combination with pembrolizumab (Keytruda) in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing: 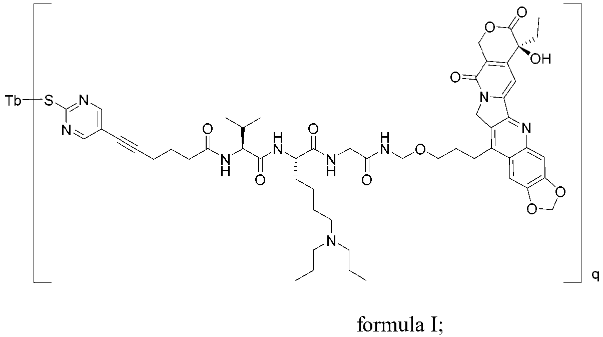
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided an anti-B7H3 antibody-drug conjugate for use in combination with pembrolizumab (Keytruda) and a platinum-based drug in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing: 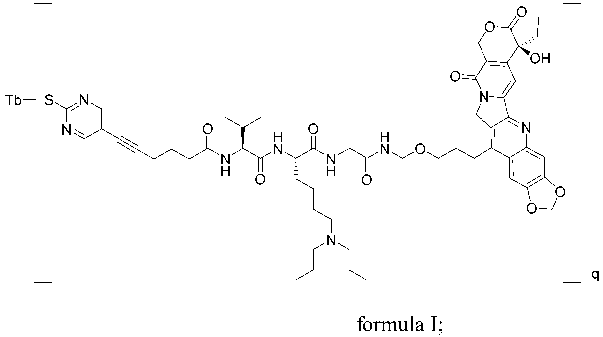
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided a PD-1 antibody for use in combination with an anti-B7H3 antibody-drug conjugate in treating cancer, wherein the PD-1 antibody is pembrolizumab (Keytruda), and the anti-B7H3 antibody-drug conjugate is an antibody-drug conjugate having the structure shown in formula I, a pharmaceutically acceptable salt, stereoisomer or metabolite thereof, or a solvate of any of the foregoing: 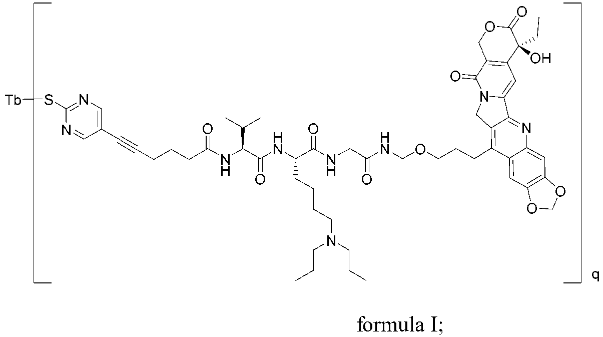
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided a PD-1 antibody for use in combination with an anti-B7H3 antibody-drug conjugate and a platinum-based drug in treating cancer, wherein the PD-1 antibody is pembrolizumab (Keytruda), and the anti-B7H3 antibody-drug conjugate is an antibody-drug conjugate having the structure shown in formula I, a pharmaceutically acceptable salt, stereoisomer or metabolite thereof, or a solvate of any of the foregoing: 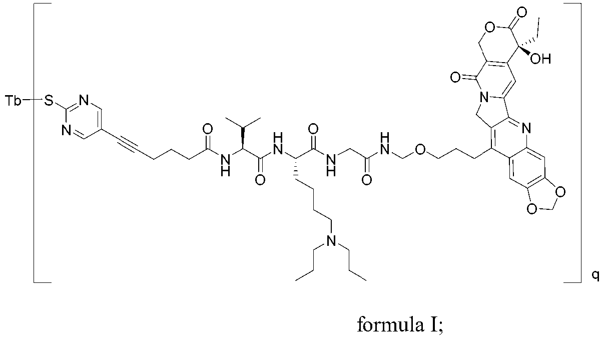
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, there is also provided a combination of an anti-B7H3 antibody-drug conjugate and pembrolizumab (Keytruda) for use in treating cancer, wherein the anti-B7H3 antibody-drug conjugate is an antibody-drug conjugate having the structure shown in formula I, a pharmaceutically acceptable salt, stereoisomer or metabolite thereof, or a solvate of any of the foregoing: 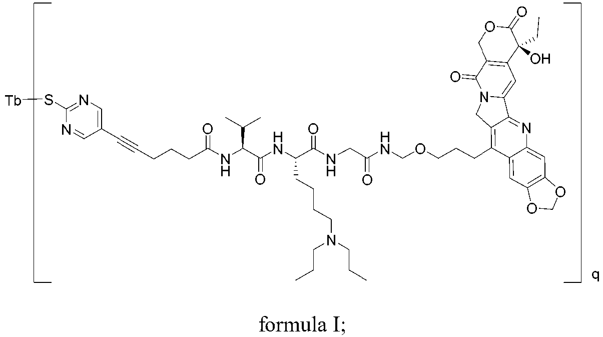
wherein S is a sulphur atom on Tb; Tb is an anti-B7H3 antibody or antigen-binding fragment thereof; q is selected from any numerical value ranging from 0.1 to 16.0; preferably, q is selected from 1, 2, 3, 4, 5, 6, 7, 8, 9, 10; more preferably, q is 2, 4, 6 or 8; still more preferably, q is 8;
the anti-B7H3 antibody or antigen-binding fragment thereof comprises a heavy chain variable region and a light chain variable region, wherein:
   the heavy chain variable region (VH) comprises:
      (i) CDR-H1 comprising the amino acid sequence set forth in SEQ ID NO: 1;
      (ii) CDR-H2 comprising the amino acid sequence set forth in SEQ ID NO: 2; and
      (iii) CDR-H3 comprising the amino acid sequence set forth in SEQ ID NO: 3; and
   the light chain variable region (VL) comprises:
      (i) CDR-L1 comprising the amino acid sequence set forth in SEQ ID NO: 4;
      (ii) CDR-L2 comprising the amino acid sequence set forth in SEQ ID NO: 5; and
      (iii) CDR-L3 comprising the amino acid sequence set forth in SEQ ID NO: 6;
   wherein the CDRs are determined in accordance with the Kabat definition scheme.

In some embodiments, treatment with a combination of an anti-B7H3 antibody-drug conjugate and an immune checkpoint inhibitor has a significant inhibitory effect on tumours. In a mouse tumour-bearing model, compared with monotherapy, combined administration of the anti-B7H3 antibody-drug conjugate and an anti-PD-1 antibody can synergistically inhibit tumour growth and produce a significant antitumour effect.

In some embodiments, the present disclosure further experimentally verifies the inhibitory effect of the combination of the anti-B7H3 antibody-drug conjugate and the anti-PD-1 antibody and/or the chemotherapeutic drug on tumours. Not limited to verification methods of animal tumour models, in vitro cell models, human clinical trials, and the like are also included; tumour types are not limited to colorectal cancer, but may also be other tumours, including but not limited to nasopharyngeal carcinoma, non-small cell lung cancer, small cell lung cancer, oesophageal squamous cell carcinoma, prostate cancer, head and neck squamous cell carcinoma, and the like.

### DETAILED DESCRIPTION OF THE INVENTION

Unless defined otherwise, all terms used herein have the same meanings as commonly understood by those of ordinary skill in the art. For related definitions and terms, reference may be made, for example, to Current Protocols in Molecular Biology (Ausubel). Meanwhile, for a better understanding of the present disclosure, definitions and explanations of relevant terms are provided below.

All documents mentioned herein are incorporated herein by reference in their entirety.

The B7H3 antibodies with native sequences herein may be isolated from nature, or may be prepared by recombinant DNA techniques, chemical synthesis methods, or combinations thereof.

As used herein, the term "antibody" is used in its broadest sense and comprises intact monoclonal antibodies, polyclonal antibodies and multispecific antibodies formed from at least two intact antibodies (e.g., bispecific antibodies), so long as they have desired biological activity.

As used herein, the term "monoclonal antibody" refers to an antibody from a population of substantially homogeneous antibodies, i.e. all antibodies constituting the population are identical except for a small number of natural mutations that may be present. Monoclonal antibodies have high specificity to one determinant (epitope) of an antigen, whereas polyclonal antibodies opposite thereto comprise different antibodies directed against different determinants (epitopes). In addition to specificity, an advantage of monoclonal antibodies also lies in the fact that they may be synthesised free from contamination by other antibodies. The modifier "monoclonal" here represents that the antibody is characterized by being from a substantially homogeneous population of antibodies, and is not to be construed as requiring preparation by any particular method.

As used herein, monoclonal antibodies also specifically comprise chimeric antibodies, that is, part of the heavy chain and/or light chain is identical or homologous to some, certain class of or certain subclass of antibodies, while the remainder is identical or homologous to some other, another class of or another subclass of antibodies, as long as they have the desired biological activity (see, for example, US4,816,567; and Morrison et al., 1984, PNAS, 81: 6851-6855). Chimeric antibodies that may be used in the present invention comprise primatised antibodies, which comprise variable region antigen-binding sequences from non-human primates (e.g., Old World monkeys, orangutans, and the like) and human constant region sequences.

As used herein, "a plurality" comprises more than one choice, for example, two, three, four, or more choices. In a particular embodiment, "a plurality" represents at least two.

The term "antibody fragment" refers to a portion of an antibody, preferably the antigen-binding region or variable region. Examples of antibody fragments comprise Fab, Fab', F(ab')₂ and Fv fragments; diabodies; linear antibodies; and single-chain antibody molecules.

In the present disclosure, the term "drug" refers to a substance that inhibits or prevents the function of cells and/or causes cell death or destruction.

The antibody-drug conjugate of the present invention may be in the form of a pharmaceutically acceptable salt, or in the form of a stereoisomer, or in the form of a metabolite, or in the form of a solvate, and the salt, stereoisomer or metabolite may also be in the form of a solvate.

The term "pharmaceutically acceptable salt" refers to salts that retain the bio-availability and properties of a compound, and they are biologically or otherwise desirable for use as a drug. In many cases, the antibody-drug conjugate of the present invention may form acid addition salts and/or base addition salts by virtue of amino groups and/or carboxyl groups or similar groups present therein.

The pharmaceutically acceptable acid addition salts may be salts formed with inorganic acids or organic acids. The inorganic acids comprise, for example, hydrochloric acid, hydrobromic acid, sulphuric acid, nitric acid and phosphoric acid, and the like. The organic acids comprise, for example, acetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulphonic acid, ethanesulphonic acid, p-toluenesulphonic acid and salicylic acid, and the like.

Pharmaceutically acceptable base addition salts may be salts formed with inorganic bases or organic bases. The salts formed with inorganic bases comprise, for example, sodium salts, potassium salts, lithium salts, ammonium salts, calcium salts, magnesium salts, iron salts, zinc salts, copper salts, manganese salts and aluminium salts, and the like, particularly preferably, ammonium salts, potassium salts, sodium salts, calcium salts and magnesium salts. The organic bases comprise, for example, primary amines, secondary amines and tertiary amines, substituted amines (including naturally occurring substituted amines), cyclic amines, basic ion exchange resins, and the like. Specific examples of organic bases are isopropylamine, trimethylamine, diethylamine, N-ethylethylamine, tripropylamine and ethanolamine.

Pharmaceutically acceptable salts can be obtained using standard procedures well known in the art, for example, by reacting a sufficient quantity of basic compounds with a suitable acid which provides pharmaceutically acceptable anions.

The term "stereoisomer" represents an isomer formed due to at least one asymmetric centre. In a compound having one or more asymmetric centres, it can produce racemates, racemic mixtures, single enantiomers, diastereomeric mixtures and individual diastereomers. Particular individual molecules may also be present as geometric isomers (cis/trans). Unless otherwise indicated, when the stereochemistry of the disclosed compound is not explicitly specified in the name or structure of the disclosed compound and the disclosed compound has one or more asymmetric centres, it should be understood to represent all possible stereoisomers of the compound.

The term "solvate" refers to a solvate formed by association of one or more solvent molecules with an antibody-drug conjugate of any formula (I) or a pharmaceutically acceptable salt or isomer thereof. The term solvate includes hydrates (for example, hemihydrate, monohydrate, dihydrate, trihydrate, tetrahydrate and similar hydrates).

Also included within the scope of the present disclosure are metabolites of the compounds of the present invention, that is, substances formed in vivo when the compounds of the present invention are administered. Such products can result from, for example, oxidation, reduction, hydrolysis, amidation, deamidation, esterification, de-esterification, enzymatic cleavage, and the like, of the administered compound. Accordingly, the present invention includes metabolites of the compounds of the present invention, including compounds produced by a method of contacting a compound of the present invention with a mammal for a time sufficient to produce a metabolite thereof.

As used herein, the term "treatment" refers to a method implemented in order to obtain a beneficial or desired clinical result. For purposes of the present invention, beneficial or desired clinical results include, but are not limited to, alleviating symptoms, reducing disease lesions, stabilising (that is, no longer worsening) the state of disease, delaying or slowing the progression of disease, ameliorating or alleviating the state of disease, and relieving symptoms (whether partially or completely), whether detectable or undetectable. Furthermore, "treatment" may also refer to prolongation of survival relative to expected survival (if not receiving treatment).

As used herein, the term "progression of a disease" or "disease progression" refers to an increase of at least 20% in the sum of diameters of target lesions, taking as reference the smallest sum of diameters recorded for all target lesions throughout the study (including the baseline sum if that is the smallest value), together with an absolute increase of at least 5 mm in the sum of diameters; alternatively, disease progression may be indicated by the appearance of one or more new lesions.

As used herein, the term "therapeutically effective amount" or "effective amount" refers to an amount of an anti-B7H3 antibody-drug conjugate (anti-B7H3-ADC) and/or an anti-PD-1 antibody or antigen-binding fragment thereof and/or a chemotherapeutic drug that is effective, when administered to a cell, tissue or subject being treated, in preventing or slowing the disease or condition to be treated. A therapeutically effective dose further refers to an amount of an anti-B7H3 antibody-drug conjugate (anti-B7H3-ADC) and/or an anti-PD-1 antibody or antigen-binding fragment thereof and/or a chemotherapeutic drug sufficient to produce alleviation of symptoms, for example including treatment, cure or alleviation of the relevant medical condition, or improvement in the rate of treatment, cure, or alleviation of the condition. The effective amount for a specific subject being treated may vary depending on a variety of factors, such as the disease to be treated, the overall health status of the patient, the method, route and dosage of dosing, and the severity of side effects. An effective amount may be the maximum dose or dosing regimen that avoids significant side effects or toxic effects. A therapeutically effective amount will typically alleviate symptoms by at least 10%, typically by at least 20%, by at least about 30%, by at least 40%, or by at least 50%.

As used herein, the terms "individual", "subject" or "patient" refer to any animal that is as the target of treatment, observation or experimentation, including (but not limited to) non-human primates, rodents, and the like. Typically, when referring to a human individual, the terms "subject" and "patient" are used interchangeably herein. The mammal may be one or more selected from humans, bovines (for example, cattle), pig-like animals (for example, pigs), sheep-like animals (for example, sheep), goat-like animals (for example, goats), equines (for example, horses), canines (such as domestic dogs), felines (such as domestic cats), lagomorphs (such as rabbits), rodents (such as rats or mice), and the like. In particular embodiments, the subject is a human.

As used herein, the terms "complete response (CR)", "partial response (PR)", "stable disease (SD)", "progressive disease (PD)", "objective response rate (ORR)", "disease control rate (DCR)" are defined according to the following methods:
When the disease is a solid tumour, the efficacy for solid tumours may be evaluated as "complete response (CR)", "partial response (PR)", "stable disease (SD)", "progressive disease (PD)", "objective response rate (ORR)", "disease control rate (DCR)" according to the following criteria (see New response evaluation criteria in solid tumours: Revised RECIST guideline (version 1.1), E.A. Eisenhauer et al., "EUROPEAN JOURNAL OF CANCER", 45 (2009), pp. 228-247). The evaluation of target lesions is specifically as follows:
Complete response (CR): all target lesions disappear, the short diameters of all pathological lymph nodes (including target nodes and non-target nodes) must be reduced to <10 mm, and there are no new lesions.

Partial response (PR): the sum of the diameters of target lesions (the short diameters are taken for lymph nodes) is reduced by at least 30% compared with the baseline level. There are no obvious progression of non-target lesions and no new lesions.

Stable disease (SD): the degree of reduction in target lesions does not reach PR, the degree of increase also does not reach the PD level, the response therefor lies between the PR and the PD level, and the smallest value of the sum of the diameters during the study may be used as a reference.

Progressive disease (PD): taking the smallest value of the sum of the diameters of all measured target lesions as a reference during the experimental study process, the sum of the diameters is relatively increased by at least 20% (the baseline value is taken as a reference if the baseline measured value is the smallest); in addition, the absolute value of the sum of the diameters of the measured target lesions must increase by at least 5 mm (the presence of one or more new lesions is also considered as the progressive disease).

Objective response rate (ORR): it refers to the proportion of patients whose tumours have shrunk to a defined extent, including cases of CR and PR. Subjects must have measurable tumour lesions at baseline, and the efficacy evaluation criteria is according to RECIST 1.1.

Disease control rate (DCR): the percentage of patients who achieved complete response (CR) and partial response (PR) as well as stable disease (SD) among the total number of participants in the analysis.

PFS and OS can be measured according to the criteria set according to the National Cancer Institute and the U.S. Food and Drug Administration with respect to new drug approval. See Johnson et al., J. Clin. Oncol. 21(7):1404-1411 (2003).

Progression-free survival (PFS) refers to the time from enrollment to disease progression or death. PFS is generally measured using the Kaplan-Meier method and the Response Evaluation Criteria in Solid Tumours (RECIST) 1.1 criteria. Generally, progression-free survival refers to the state where the patient remains alive and the cancer has not worsened.

"Overall survival" (OS) refers to the time from enrollment of a patient to death or to the date on which the patient is last known to be alive. OS comprises an extension of the expected life span compared with untreated or non-treated individuals or patients. Overall survival refers to the state where the patient remains alive for a specified period of time, for example one year, five years, etc. from diagnosis or treatment. In a patient population, overall survival is measured by the median overall survival (mOS).

Depth of response (DpR): the depth of response refers to the degree of reduction in tumour volume or disease burden during treatment, and specifically refers to the percentage of maximum tumour shrinkage achieved during treatment compared to the baseline; if CR occurs, DpR is 100. It is usually measured on the basis of imageology or clinical assessment.

Duration of response (DoR): the duration of response refers to the duration of the tumour response state after treatment, and specifically refers to the time from the time when subjects who have achieved a confirmed complete response (CR) or a confirmed partial response (PR) achieve the confirmed complete response (CR) or the confirmed partial response (PR) for the first time to the time of disease progression for the first time.

Time to response (TTR): the time to response refers to the time from the start of treatment to the time when a patient achieves a response (CR or PR) state for the first time.

### BRIEF DESCRIPTION OF THE DRAWINGS

Figure 1. Efficacy testing results of an antibody-drug conjugate (ADC1) in combination with an anti-PD-1 antibody in an MC38-B model
Figure 2. Body weight in the MC38-B model treated with an antibody-drug conjugate (ADC1) in combination with an anti-PD-1 antibody
Figure 3. Efficacy testing results of an antibody-drug conjugate (ADC1) in combination with an anti-PD-1 antibody in the MC38-B recurrence model
Figure 4. Body weight in the MC38-B recurrence model treated with an antibody-drug conjugate (ADC1) in combination with an anti-PD-1 antibody

### DETAILED DESCRIPTION OF EMBODIMENTS

### Sequence information

The sequence information involved in the present application is described in the table below:

| SEQ ID NO | Name | Sequence |
|---|---|---|
| 1 | CDR-H1 | SYAMN |
| 2 | CDR-H2 | GISGSGGSTYYADSVQG |
| 3 | CDR-H3 | ARSGYDYFFDY |
| 4 | CDR-L1 | RASQRISSSFLA |
| 5 | CDR-L2 | GASSRAT |
| 6 | CDR-L3 | QQYSNSPLT |
| 7 | Heavy chain variable region (VH) | EVQLLESGGGLVQPGGSLRLSCAASGFTFSSYAMNWVRQAPGKGLEWV SGISGSGGSTYYADSVQGRFTISRDNSKNTLYLQMNSLRAEDTAVYYCAK ARSGYDYFFDYWGQGTLVTVSS |
| 8 | Light chain variable region (VL) | EIVLTQSPGTLSLSPGERATLSCRASQRISSSFLAWYQQKPGQAPSLLIYGA SSRATGIPDRFSGSGSGTDFTLTISSLEPEDFAVFYCQQYSNSPLTFGGGTK VEIK |
| 9 | Heavy chain (HC) | EVQLLESGGGLVQPGGSLRLSCAASGFTFSSYAMNWVRQAPGKGLEWV SGISGSGGSTYYADSVQGRFTISRDNSKNTLYLQMNSLRAEDTAVYYCAK ARSGYDYFFDYWGQGTLVTVSSASTKGPSVFPLAPSSKSTSGGTAALGCL VKDYFPEPVTVSWNSGALTSGVHTFPAVLQSSGLYSLSSVVTVPSSSLGT QTYICNVNHKPSNTKVDKKVEPKSCDKTHTCPPCPAPELLGGPSVFLFPP KPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQ PREPQVYTLPPSRDELTKNQVSLTCLVKGFYPSDIAVEWESNGQPENNYK TTPPVLDSDGSFFLYSKLTVDKSRWQQGNVFSCSVMHEALHNHYTQKSL SLSPGK |
| 10 | Light chain (LC) | EIVLTQSPGTLSLSPGERATLSCRASQRISSSFLAWYQQKPGQAPSLLIYGA SSRATGIPDRFSGSGSGTDFTLTISSLEPEDFAVFYCQQYSNSPLTFGGGTK VEIKRTVAAPSVFIFPPSDEQLKSGTASVVCLLNNFYPREAKVQWKVDNA LQSGNSQESVTEQDSKDSTYSLSSTLTLSKADYEKHKVYACEVTHQGLSS PVTKSFNRGEC |

The present disclosure is further illustrated through description of the specific embodiments below, but this is not intended to limit the present disclosure. Those skilled in the art may make various modifications or improvements in light of the teachings of the present disclosure without departing from the basic spirit and scope of the present disclosure.

### Example 1: Production of anti-B7H3 antibody-drug conjugate

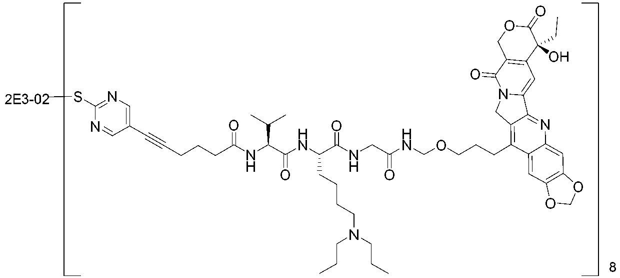

Referring to the production method described in Example 4.1.7.1 of the International Publication No. WO2022170971A1, an anti-B7H3 ADC composition (hereinafter referred to as "ADC1") was produced using a humanised anti-B7H3 antibody (2E3-02).

### Example 2: Efficacy testing of antibody-drug conjugate (ADC1) in combination with an anti-PD-1 antibody in the MC38-B transplanted tumour

### 1. Experimental Materials

Test compounds: ADC1, anti-PD-1, ADC 1+anti-PD-1, and saline as the negative control. The anti-PD-1 is a murine anti-mouse PD-1 antibody expressed in cells constructed on the basis of the heavy-chain and light-chain sequences of antibody C disclosed in patent WO2017132827A1.
Experimental cells: MC38-B7H3 cells (hereinafter referred to as MC38-B, MC38 cells transfected with the human B7H3 recombinant protein);
Experimental animals: female C57BL/6J mice, 5-6 weeks old, purchased from GemPharmatech Co., Ltd.

### 2. Experimental Regimen

### 2.1. Cell Treatment

MC38-B cells were cultured with 1640 medium containing 10% FBS in 15 cm culture dishes. When the cells reached approximately 80% to 90% confluence, they were digested with trypsin-EDTA, washed twice with PBS, centrifuged and resuspended in pre-cooled PBS, counted using a cell counter, and diluted with PBS to a cell concentration of 5 × 10⁷/ml.

### 2.2. Tumour cell transplantation

C57BL/6J mice were acclimatised to the laboratory environment for 2 to 5 days. MC38-B cells were then inoculated subcutaneously into the right flank at 5 × 10⁶ cells per mouse in a volume of 0.1 mL. The experiment was initiated when the tumours reached approximately 150 mm³.

### 2.3. Animal Dosing and Detection

### 2.3.1 Drug combination experimental protocol

The enrolled tumour-bearing mice were dosed according to the following protocol:

**Table 1. Dosing protocol**

| Serial Number | Group | Day 1 of dosing | Day 4 of dosing | Day 8 of dosing | Day 11 of dosing | Day 15 of dosing | Day 17 of dosing | Day 18 of dosing | Dosage |
|---|---|---|---|---|---|---|---|---|---|
| 1 | Vehicle | Saline | Saline | Saline | Saline | Saline | / | Saline | / |
| 2 | Test Group 1 | ADC1 | ADC1 | ADC1 | ADC1 | / | ADC1 | | 8 mg/kg |
| 3 | Test Group 2 | Anti-PD-1 | Anti-PD-1 | Anti-PD-1 | Anti-PD-1 | / | Anti-PD-1 | / | 2 mg/kg |
| 4 | Test Group 3 | ADC1+ Anti-PD-1 | ADC1+ Anti-PD-1 | ADC1+ Anti-PD-1 | ADC1+ Anti-PD-1 | / | ADC1+ Anti-PD-1 | / | 8+2 mg/kg |

| | | | | | | | | | |
|---|---|---|---|---|---|---|---|---|---|
| Note: There were 5 animals in each group, and the dosing volume was 10 mL/kg. | | | | | | | | | |

### 2.3.2 Recurrence model test protocol

MC38-B cells were re-inoculated into test group 3 (i.e. the ADC1 + anti-PD-1 drug combination) on Day 29, which was designated as Day 0.

For the control group (saline group), newly enrolled naive mice matched for body weight and weeks of age were inoculated with MC38-B cells according to the protocol in Section 2.2.

In the above two groups, only tumour size was measured, and no treatment was administered.

### 2.4. Measurement of tumour volume and body weight:

Tumour volume and body weight were measured twice weekly, and T/C (%) and TGI (%) were calculated. The formulae were as follows: relative tumour proliferation rate, T/C (%) = TRTV/CRTV × 100% (TRTV: mean RTV of the treatment group; CRTV: mean RTV of the control group; RTV = Vt/V0, where V0 is the tumour volume of the animal at the time of grouping and Vt is the tumour volume of the animal after treatment); and relative tumour growth inhibition rate, TGI (%) = (1 - T/C) × 100% (where T and C are the relative tumour volumes (RTV) of the treatment group and the control group, respectively, at a specific time point).

The formula for calculating the tumour volume (V): V = 1/2 × Llong × Lshort², wherein Llong and Lshort represent the long diameter and the short diameter of the tumour, respectively.

### 3. Experimental Results

### 3.1 Drug combination experimental results

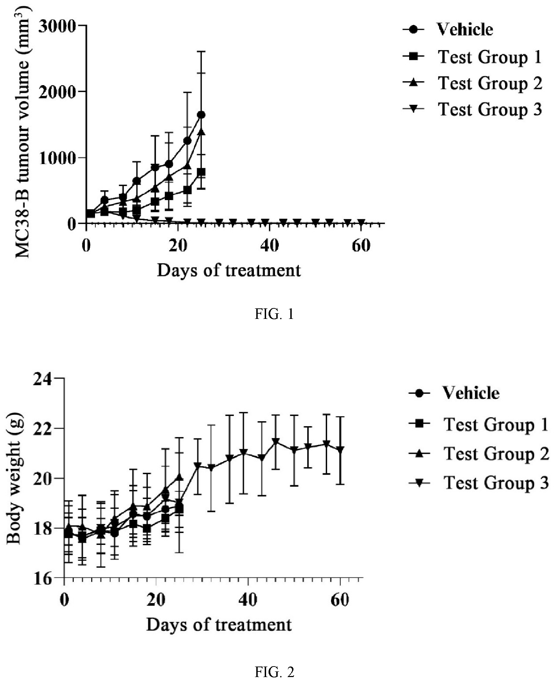

The detailed results are shown in Table 2 as well as Figure 1 and Figure 2. The testing results indicated that the tumour inhibition rates of Test Group 1, Test Group 2, and Test Group 3 on Day 25 of dosing were 52.58%, 15.05%, and 99.49%, respectively. The TGIs of the monotherapy groups (Test Group 1 and Test Group 2) were both less than 60%, indicating no significant efficacy, whereas the drug combination group (Test Group 3) showed a very marked tumour inhibition effect, with a TGI reaching 99.49%, and tumour regression had already occurred. Meanwhile, after completion of the fifth dosing (Day 17), the tumour volume testing time was extended to Day 60, and the tumours continued to regress. The above data indicated that, compared with the monotherapy groups, the drug combination group produced a significant efficacy improvement. Meanwhile, during the dosing period, the body weights of animals in each group increased steadily, indicating that neither monotherapy nor drug combination showed obvious toxicity.

**Table 2. Data for the MC38-B transplanted tumour model**

| | Tumour volume (mm³) | | | | | | | | T/C (%) | TGI (%) |
|---|---|---|---|---|---|---|---|---|---|---|
| Sample | Day1 | Day4 | Day8 | Day 11 | Day 15 | Day 18 | Day 22 | Day 25 | / | / |
| Saline | 150 | 354 | 399 | 642 | 850 | 903 | 1257 | 1649 | / | / |
| Test Group 1 | 150 | 177 | 173 | 198 | 330 | 416 | 503 | 782 | 47.42 | 52.58 |
| Test Group 2 | 149 | 251 | 327 | 379 | 548 | 712 | 885 | 1401 | 84.95 | 15.05 |
| Test Group 3 | 148 | 179 | 110 | 64 | 42 | 33 | 13 | 8 | 0.51 | 99.49 |

### 3.2 Tumour measurement results in the recurrence model

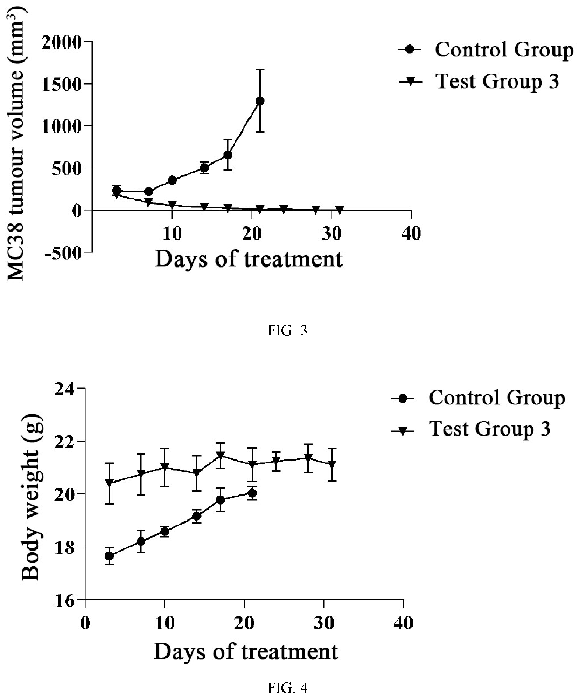

The detailed results are shown in Table 3 as well as Figure 3 and Figure 4. The tumour measurement results indicated that, after tumours were re-inoculated in Test Group 3, the TGI on Day 21 reached 99.29%, and tumour regression still occurred. However, the tumour volume of mice in the control group became excessive (>1000 mm³), and the experiment was terminated early for animal welfare reasons. The above data indicated that, after combination of ADC1 and anti-PD-1, tumour regrowth could be effectively inhibited. There was no significant decrease in terms of body weight of animals in any group.

**Table 3. Data for the recurrence model**

| | Tumour volume (mm³) | | | | | | | | | T/C (%) | TGI (%) |
|---|---|---|---|---|---|---|---|---|---|---|---|
| Group | Day 3 | Day 7 | Day 10 | Day 14 | Day 17 | Day 21 | Day 24 | Day 28 | Day 31 | / | / |
| Control Group | 235 | 222 | 355 | 503 | 658 | 1297 | / | / | / | / | / |
| Test Group 3 | 180 | 90 | 59 | 37 | 23 | 9 | 12 | 5 | 2 | 0.71 | 99.29 |

### Example 3. Study of the activity of an anti-B7H3 antibody-drug conjugate in combination with Keytruda in a tumour cell and human PBMC co-culture experiment

The in vitro activity of ADC 1 in combination with Keytruda was evaluated using the co-culture of NCI-H1975-Lux cells and human PBMCs.

Cryopreserved human PBMCs were rapidly thawed in a 37°C water bath. The cell density was adjusted to 2 × 10⁶/ml, and a medium containing SEB was added to the cell suspension to achieve a final SEB concentration of 100 ng/ml. The cells were then cultured for 72 h at 37°C and 5% CO₂ to stimulate and activate the PBMCs. After stimulation, the PBMCs were centrifuged and resuspended, and the cell density was adjusted to 2 × 10⁵/ml.

NCI-H1975-Lux cells (constructed by Crown Bioscience Inc.) were cultured adherently in RPMI 1640 medium containing 10% FBS at 37°C and 5% CO₂. Cells in the logarithmic growth phase were harvested and counted to ensure that cell viability was greater than 90%. The cell density was adjusted, and the cells were plated at 10,000 cells/100 µl/well and allowed to adhere overnight. 50 µl of PBMC suspension was added to wells containing adherent NCI-H1975-Lux cells for co-culture. According to the experimental design, to treat the cells, 0.3 µM and 0.6 µM ADC 1 and 10 µg/ml Keytruda were added, as well as 10 µg/ml hIgG4 isotype (isotype control) for the corresponding control group. The cells were further cultured for 72 h at 37°C and 5% CO₂.

After incubation, the 96-well plate was equilibrated at room temperature for 30 min. 50 µL of Steady-Glo^{®} reagent (Promega, part number E2520) was added to each well. After incubation for 20 min, the chemiluminescence value was measured to obtain the ratio for each group relative to the luminescence value of NCI-H1975-Lux cells co-cultured with human PBMCs without addition of any substance.

The results are shown in Table 4. ADC1 + Keytruda showed a lower luminescence ratio than ADC1 + hIgG4 isotype, Keytruda alone, and hIgG4 isotype alone, indicating stronger tumour-inhibitory activity. In addition, the tumour-inhibitory activity increased with increasing ADC concentration.

**Table 4. Test results of in vitro test**

| Group | ADC1 (0.6 µM) | | ADC1 (0.3 µM) | |
|---|---|---|---|---|
| ADC1 + Keytruda | | 0.472 | | 0.751 |
| ADC1 + hIgG4 isotype | | 0.681 | | 0.872 |
| Keytruda (monotherapy) | 0.890 | | | |
| hIgG4 isotype (monotherapy) | 1.049 | | | |

### Example 4: In vivo activity testing of antibody-drug conjugate (ADC) in combination with Anti-PD1 antibody

### 1. Experimental Materials

Test compounds: ADC1, anti-mouse PD-1 antibody (anti-mPD-1, BioXcell, BE0146), and saline as the vehicle control.

Experimental cells: B-hB7-H3 CT26. WT cells overexpressing human B7-H3, which were constructed by Biocytogen Pharmaceuticals (Beijing) Co., Ltd.

Experimental animals: female BALB/c mice, 6-8 weeks old, provided by Biocytogen.

### 2. Experimental Regimen

### 2.1. Cell Treatment

The culture conditions for B-hB7-H3 CT26. WT cells were: RPMI 1640 + 10% FBS, cultured in an incubator at 37°C, 5% CO₂. Cells were subjected to conventional digestive treatment and passage twice weekly using trypsin containing EDTA. The cells were washed twice with PBS, and then were centrifuged and resuspended in pre-chilled PBS. Cells were counted using a cell counter, the cell suspension was adjusted to an appropriate concentration, and the cells were inoculated.

### 2.2. Tumour Cell Inoculation

BALB/c mice were acclimatised to the laboratory environment for 3-5 days. Cells were then subcutaneously inoculated into the right dorsal flank of each mouse at 1×10⁶ cells per 0.1 mL PBS. Grouping and dosing were initiated when the mean tumour volume reached about 80-120 mm³.

### 2.3. Animal Dosing and Detection

The enrolled tumour-bearing nude mice were dosed according to the regimen shown in Table 5 below:

**Table 5. Dosing regimen**

| Group | Dosing group | Dose (mg/kg) | Method of dosing | | Dosing cycle | |
|---|---|---|---|---|---|---|
| 1 | Saline | 3 | | i.v. | | BIW * 4 |
| 2 | ADC1 + anti-mPD-1 | ADC1 3 mg/kg, anti-mPD-1 15 mg/kg | | i.v. | | BIW * 4 |

### 2.4. Measurement of Tumour Volume and Body Weight

The day of the first dosing was designated Day 0. A total of four doses were administered, and the diameter of the tumour and body weight were measured regularly. Tumour volume, relative tumour proliferation rate, and relative tumour inhibition rate were calculated, and tumour growth curves were plotted. The formulae were as follows:
The tumour volume (V) was calculated as: V = 1/2 × Llong × Lshort2, wherein Llong and Lshort represent the long diameter and short diameter of the tumour, respectively.

Relative tumour growth inhibition rate, TGI (%), was calculated as follows: TGI (%) = [1 - (mean tumour volume of the treatment group on a given day - mean tumour volume of the treatment group at the start of dosing) / (mean tumour volume of the vehicle control group on the same day - mean tumour volume of the vehicle control group at the start of dosing)] × 100%.

Relative tumour proliferation rate, T/C (%): the calculation formula is as follows: T/C (%) = TRTV/CRTV × 100% (TRTV: RTV of the treatment group; CRTV: RTV of the vehicle control group). RTV (relative tumour volume): the calculation formula is RTV = Vt/V0, wherein V0 is the mean tumour volume obtained by measurements at the time of dosing after grouping (i.e. D0), Vt is the mean tumour volume at a certain measurement, and TRTV and CRTV take data of the same day.

### 3. Experimental Results

The testing results indicated that the antibody-drug conjugate of the present disclosure in combination with the anti-PD 1 exhibited a relatively significant tumour inhibition effect. During the dosing period, there was no significant reduction in body weight or significant drug toxicity of animals in each group. Specific results are shown in Table 6.

**Table 6. Tumour volume data of B-hB7-H3 CT26.WT model**

| | Average tumour volume (mm³) | | | | | TGI (%) D14 |
|---|---|---|---|---|---|---|
| Group | D0 | D4 | D7 | D11 | D14 | |
| 1 | 92 | 164 | 340 | 566 | 773 | / |
| 2 | 92 | 174 | 219 | 173 | 142 | 92.7% |

### Example 5. Clinical study of an anti-B7H3 antibody-drug conjugate in combination with immunotherapy for multiple advanced solid tumours

This clinical study is a multicentre, open-label, phase I study evaluating the safety, efficacy, and pharmacokinetics of the anti-B7H3 antibody-drug conjugate of the present invention in combination with serplulimab, with or without platinum-based drugs, in selected patients with advanced solid tumours.

The selection criteria for subjects were as follows:
The inclusion criteria were as follows:
1) Before initiation of the study, the subject had been informed of the study and had voluntarily signed and dated the informed consent form (ICF).
2) Age ≥18 years and ≤75 years.
3) Enrolled population (staged according to the 8th edition of the Union for International Cancer Control [UICC] and American Joint Committee on Cancer [AJCC] staging system):
   Dose escalation: histologically or cytologically confirmed advanced solid tumours that had progressed after standard treatment, were intolerant to standard treatment, or had no available standard treatment.

### Cohort expansion:

### Cohort A:

a) Histologically or cytologically confirmed metastatic (stage IVB) or recurrent NPC;
b) Prior receipt of platinum-containing chemotherapy;
c) Patients were permitted to have previously received anti-PD-(L)1 therapy.
d) Disease progression or intolerance was documented during or after the most recent systemic treatment.

### Cohort B:

a) Histologically or cytologically confirmed metastatic (stage IVB) or recurrent NPC;
b) No previous systemic treatment for the current disease. If the patient had received chemoradiotherapy with curative intent before diagnosis of metastatic or recurrent disease, and there was a treatment-free interval of at least 6 months between the last chemoradiotherapy and the diagnosis of metastatic or recurrent disease, then the patient could be enrolled.

### Cohort C:

a) Histologically or cytologically confirmed extensive-stage SCLC (AJCC 8th edition stage IV [any T, any N, Mla/b/c], or T3-4 disease that could not be included in a tolerable radiotherapy plan because of multiple pulmonary nodules or excessively large tumour/nodule volume);
b) No previous systemic treatment for the current disease. If the patient had previously received chemoradiotherapy with curative intent for limited-stage disease, and there was a treatment-free interval of at least 6 months between the last chemoradiotherapy and the diagnosis of extensive-stage SCLC, then the patient could be enrolled.

### Cohort D:

a) Histologically or cytologically confirmed locally advanced (stage IIIB/IIIC) or metastatic (stage IV) NSCLC that could be unsuitable for curative surgery or radiotherapy;
b) No known epidermal growth factor receptor (EGFR) sensitising mutations, anaplastic lymphoma kinase (ALK) and c-ros proto-oncogene (ROS1) fusions. Patients with a pathological pattern of adenocarcinoma or mixed with an adenocarcinoma component must undergo the corresponding gene detection. Patients with a pathological pattern of squamous cell carcinoma or other pathological types do not require the corresponding gene detection;
c) Prior receipt of no more than two lines of systemic treatment, including platinum-containing chemotherapy;
d) Patients were permitted to have previously received anti-PD-(L)1 therapy.
e) Disease progression or intolerance was documented during or after the most recent systemic treatment.

### Cohort E:

a) Histologically or cytologically confirmed locally advanced (stage IIIB/IIIC) or metastatic (stage IV) NSCLC that could be unsuitable for curative surgery or radiotherapy;
b) No known epidermal growth factor receptor (EGFR) sensitising mutations, anaplastic lymphoma kinase (ALK) and c-ros proto-oncogene (ROS1) fusions. Patients with a pathological pattern of adenocarcinoma or mixed with an adenocarcinoma component must undergo the corresponding gene detection. Patients with a pathological pattern of squamous cell carcinoma or other pathological types do not require the corresponding gene detection;
c) No previous systemic treatment for the current disease. If the patient had previously received treatment with curative intent (not limited to surgery or adjuvant therapy), a treatment-free interval of at least 6 months was required.

### Cohort F:

a) Histologically or cytologically confirmed advanced solid tumours (excluding the tumour types included in Cohorts A-E);
b) Prior receipt of no more than two lines of systemic treatment for the current disease, or no available standard treatment regimen;
c) Disease progression or intolerance was documented during or after the most recent systemic treatment.

4) At least 1 extracranial measurable lesion according to RECIST v1.1. Lesions previously receiving radiotherapy or other local treatment may not be used as target lesions unless they had clearly progressed.

5) Ability to provide archived or fresh tumour tissue samples. For patients who were unable to provide tumour samples or had insufficient samples, enrollment could be considered on a case-by-case basis after discussion with the sponsor.

6) Eastern Cooperative Oncology Group Performance Status (ECOG PS) of 0 or 1.

7) Body organ and bone marrow functions within 7 days before the first dosing meeting the requirements are defined as follows:
a) Haemoglobin (Hb) ≥ 9.0 g/dL (without transfusion or erythropoietin treatment within 14 days before the first dosing);
b) Absolute neutrophil count (ANC) ≥ 1.5 × 10⁹/L (without granulocyte colony-stimulating factor or granulocyte-macrophage colony-stimulating factor treatment within 14 days before the first dosing);
c) Platelet count (PLT) ≥ 100 × 10⁹/L (without platelet transfusion, thrombopoietin, or interleukin-11 treatment within 14 days before the first dosing);
d) TBIL ≤ 1.5 × ULN in the absence of obvious liver metastases, or ≤ 3 × ULN in the presence of liver metastases;
e) ALT and AST ≤ 3 × ULN in the absence of obvious liver metastases, or ≤ 5 × ULN in the presence of liver metastases;
f) Serum albumin ≥ 3.0 g/dL;
g) Creatinine clearance ≥ 50 mL/min (calculated according to the Cockcroft-Gault formula);
h) Activated partial thromboplastin time (APTT) and international normalised ratio (INR) ≤ 1.5 × ULN, except for patients receiving anticoagulant therapy; such patients must have a stable anticoagulant regimen, and APTT and INR must be within the therapeutic range deemed appropriate by the investigator.

8) Female patients of childbearing potential must agree to use highly effective contraceptive measures from screening throughout the study period and for at least 6 months after the last dosing of the study drug. Male patients must agree to use highly effective contraceptive measures from screening throughout the study period and for at least 6 months after the last dosing of the study drug.

9) Expected survival ≥ 3 months.

10) Able and willing to comply with the visits and procedures specified in the study protocol.

### Exclusion criteria are as follows:

1) Mixed SCLC.
2) Suitable for local radical treatment.
3) Prior receipt of B7H3-targeted drug therapy (including antibodies, antibody-drug conjugates [ADCs], chimeric antigen receptor T cells [CAR-T], and other drugs).
4) Prior receipt of a topoisomerase I inhibitor or ADC treatment comprising a topoisomerase I inhibitor.
5) Previously experienced CTCAE Grade ≥ 3 immune-related adverse events (irAEs) during treatment with anti-PD-(L)1 therapy or other immune checkpoint inhibitors, immune checkpoint agonists, immune cell therapy, or other therapies targeting tumour immune mechanisms, except for endocrine disorders such as hyperglycaemia and hypothyroidism that had recovered to Grade ≤ 2.
6) Toxicities from prior anticancer therapy had not resolved, defined as toxicities that had not resolved to NCI CTCAE Grade ≤ 1, baseline level, or the level specified in the inclusion/exclusion criteria (except for alopecia and pigmentation). For patients with chronic Grade 2 toxicity, if there was no symptom or adequate control could be achieved with stable medication, enrollment could be considered after discussion with the sponsor.
7) Concurrent enrollment in another clinical study, unless it was an observational (non-interventional) clinical study or the patient was in the follow-up period of an interventional study.
8) Insufficient washout period from prior anticancer therapy before the first dosing of the study drug, defined as follows:
   a) Chemotherapy or small-molecule targeted therapy < 2 weeks or 5 half-lives, whichever is shorter
   b) Antibody therapy < 3 weeks;
   c) Hormonal therapy < 3 weeks;
   d) Chinese herbal medicine therapy with antitumour indications < 2 weeks;
   e) Brain radiotherapy < 2 weeks;
   f) Palliative radiotherapy < 2 weeks, and radical radiotherapy < 4 weeks.
9) Receipt of major surgery (excluding diagnostic surgery) or serious trauma within 4 weeks before the first dosing of the study drug, or being in the recovery period and judged by the investigator as likely to affect the study, or being expected to undergo major surgery during the study.
10) Prior receipt of an allogeneic stem cell or solid organ transplant.
11) Active autoimmune disease requiring systemic therapy (i.e. use of corticosteroids or immunosuppressive drugs) within 2 years before the first dosing of the study drug, or the presence of an autoimmune disease that, in the investigator's judgment, may relapse or require treatment.
12) Receipt of systemic steroids (> 10 mg/day prednisone or equivalent drugs) or other immunosuppressive therapy within 2 weeks before the first dosing of the study drug, except in the following situations:
   a) Intranasal, inhaled, topical steroids, or local steroid injection (such as intra-articular injection);
   b) Physiological doses of systemic steroids as replacement therapy (such as physiological corticosteroid replacement therapy for adrenal or pituitary insufficiency);
   c) Steroids as prophylactic medication for hypersensitivity reactions (such as allergy to CT contrast agents) or for chemotherapy-induced nausea and vomiting (CINV).
13) Receipt of any live vaccine within 4 weeks before the first dosing of the study drug, or planned receipt of a live vaccine during the study.
14) Leptomeningeal metastases or carcinomatous meningitis.
15) Brain metastases or spinal cord compression, except in the following situations:
   a) Patients with asymptomatic brain metastases who do not require immediate local or systemic therapy (such as mannitol or corticosteroids) may be permitted to be enrolled;
   b) If the patient's brain metastases have been treated and the metastasis conditions are stable (the brain imaging examination conducted at least 4 weeks before the first dosing showed that the lesions are stable, there are no new neurological symptoms, and no immediate local or systemic treatment is required within 2 weeks before the first dosing), and there is no evidence of new brain metastases or enlargement of existing brain metastases, the patient may be permitted to be enrolled.
16) Presence of uncontrolled or clinically significant cardiovascular diseases, including, but not limited to:
   a) History of symptomatic congestive heart failure (New York Heart Association [NYHA] Class II to IV), or any arterial thromboembolic event (such as myocardial infarction, unstable angina, cerebrovascular accident, or transient ischaemic attack) within 6 months before the first dosing;
   b) Receipt of percutaneous transluminal coronary angioplasty (PTCA) or coronary artery bypass graft surgery (CABG) within 6 months before the first dosing;
   c) Uncontrolled hypertension, defined as systolic blood pressure (SBP) > 160 mmHg and/or diastolic blood pressure (DBP) > 100 mmHg after antihypertensive treatment;
   d) Serious arrhythmia requiring treatment;
   e) QT interval corrected by Fridericia's formula (QTcF) is prolonged to > 450 ms in males or > 470 ms in females.
17) Presence of clinically significant concomitant pulmonary diseases, including, but not limited to:
   a) History of (non-infectious) interstitial lung disease (ILD) or interstitial pneumonitis requiring steroid treatment, or current ILD/interstitial pneumonitis;
   b) Occurrence of pulmonary embolism within 3 months before the first dosing;
   c) Prior pneumonectomy;
   d) Other moderate-to-severe pulmonary diseases that seriously affect the respiratory function and may interfere with the detection or management of drug-related pulmonary toxicities.
18) Diagnosis of Gilbert's syndrome.
19) Those accompanied with uncontrolled third-space effusion (such as pleural effusion, ascites, pericardial effusion) and requiring repeated drainage.
20) History of gastrointestinal perforation and/or fistula within 6 months before the first dosing, or active gastric and duodenal ulcer, ulcerative colitis, or other gastrointestinal diseases that, in the investigator's judgement, may cause bleeding or perforation.
21) Tumour tissue significantly invading organs adjacent to the lesions (major arteries or the trachea), resulting in a high risk of bleeding or fistula; or history of intraluminal tracheal stent implantation.
22) Severe infection (NCI CTCAE Grade ≥ 3) within 4 weeks before the first dosing, for example, those having severe pneumonia, bacteraemia, infectious complications, etc. requiring hospitalisation, or those having active infection within 2 weeks before the first dosing and requiring systemic therapy. Patients receiving prophylactic anti-infective treatment (for example, for preventing urinary tract infection or aggravation of chronic obstructive pulmonary diseases) may be eligible for enrollment after discussion with the sponsor.
23) Known human immunodeficiency virus (HIV) infection.
24) Active hepatitis B virus (HBV) or hepatitis C virus (HCV) infection. Active HBV is defined as being positive for hepatitis B core antibodies (HBcAb) and/or hepatitis B surface antigens (HBsAg), with HBV deoxyribonucleic acid (DNA) levels > 200 IU/mL or 1000 copies/mL; HBV-infected persons should receive antiviral treatment according to local treatment guidelines and be willing to continue antiviral treatment throughout the study period; active HCV is defined as being positive for hepatitis C antibodies and an HCV ribonucleic acid (RNA) level above the ULN of the study centre.
25) Any other primary malignant tumour within 3 years before the first dosing of the study drug, except for adequately resected non-melanoma skin cancer, cured carcinoma in situ, or other cured solid tumours.
26) History of severe hypersensitivity to the study drug, inactive ingredients in the formulation, or other monoclonal antibodies.
27) Lactating women, or women confirmed to be pregnant by a pregnancy test within 7 days before the first dosing.
28) Any disease/medical condition, organ system dysfunction, or social circumstance that, in the investigator's judgment, may interfere with the patient's ability to sign the informed consent, adversely affect the patient's ability to cooperate with and participate in the study, or affect the interpretation of the study results, including, but not limited to, certain severe and/or uncontrolled medical conditions (such as acute or chronic pancreatitis or immunocompromised status), psychiatric illness, and substance/alcohol abuse.

Treatment regimen: Subjects who meet all inclusion criteria and none of the exclusion criteria receive an anti-B7H3 antibody-drug conjugate (a lyophilised injection prepared according to routine methods in the art, at a dosage of 1.2, 1.6, 1.8, 2.0, or 2.4 mg/kg) and serplulimab (3.0 mg/kg or 4.5 mg/kg) in combination with or not in combination with cisplatin (70 mg/m²), by intravenous infusion. The treatment cycle is every three weeks (with or without cisplatin) or every two weeks (not in combination with cisplatin). Efficacy is assessed at each time until the disease progression, intolerable toxicity, or voluntary withdrawal occurs. The study endpoint criteria included safety and tolerability, MTD and RDE, ORR, DCR, pharmacokinetics, immunogenicity, efficacy-related biomarkers, and the like.

### Therapeutic effect:

A total of 14 patients with advanced solid tumours who had failed standard treatment were enrolled in the dose-escalation phase of this clinical study, 8 of them were subjects with evaluable efficacy and the remaining 6 subjects had not yet undergone efficacy assessment because the tumour assessment window had not yet been reached. According to the RECIST v1.1 tumour efficacy assessment criteria, among the 8 subjects evaluable for efficacy, 2 subjects achieved a confirmed partial response (PR) as the best overall response, 2 subjects achieved a partial response (PR) as the best overall response, and 4 subjects achieved stable disease (SD) as the best overall response. The detailed efficacy for the 8 efficacy-evaluable subjects is shown in Example 6 below.

### Example 6: Detailed efficacy of an anti-B7H3 antibody-drug conjugate in combination with serplulimab in patients with various advanced solid tumours

1. Subject 101001: male, 68 years old, pathologically diagnosed with non-small cell lung cancer - adenocarcinoma, with lymph node metastasis and brain metastasis. The target lesions were located in the inferior lobe of right lung and the subcarinal lymph node. After 18 weeks of treatment with an anti-B7H3 antibody-drug conjugate in combination with serplulimab, the lesion in the inferior lobe of right lung decreased from 30 mm to 11 mm, representing a reduction of 63.3%, and the subcarinal lymph node lesion decreased from 23 mm to 11 mm, representing a reduction of 52.2%. Upon assessment, the best overall efficacy reached confirmed PR.
2. Subject 101002: male, 52 years old, pathologically diagnosed with non-small cell lung cancer - squamous cell carcinoma, with pleural metastasis. The target lesions were located in the inferior lobe of left lung, the right hilar lymph node, and the left hilar lymph node. After 18 weeks of treatment with an anti-B7H3 antibody-drug conjugate in combination with serplulimab, the lesion in the inferior lobe of left lung decreased from 34 mm to 28 mm, representing a reduction of 17.6%; the lesion in the right hilar lymph node decreased from 22 mm to 18 mm, representing a reduction of 9.1%; and the lesion in the left hilar lymph node decreased from 20 mm to 16 mm, representing a reduction of 20%. Upon assessment, the best overall efficacy reached SD.
3. Subject 101004: male, 46 years old, pathologically diagnosed with small cell lung cancer, with lymph node metastasis, pleural metastasis, pleural effusion, and pericardial effusion. The target lesions were located in the left hilar region, the prevascular lymph node, and the subcarinal lymph node. After 12 weeks of treatment with an anti-B7H3 antibody-drug conjugate in combination with serplulimab, the lesion in the left hilar decreased from 93 mm to 51 mm, representing a reduction of 45.2%; the lesion in the prevascular lymph node decreased from 21 mm to 8 mm, representing a reduction of 61.9%; and the lesion in the subcarinal lymph node decreased from 24 mm to 14 mm, representing a reduction of 41.7%. Upon assessment, the best overall efficacy reached PR.
4. Subject 101006: male, 69 years old, pathologically diagnosed with non-small cell lung cancer - adenocarcinoma, with lymph node metastasis, brain metastasis, and bone metastasis. The target lesion was located in the inferior lobe of left lung. After 12 weeks of treatment with an anti-B7H3 antibody-drug conjugate in combination with serplulimab, the lesion in the inferior lobe of left lung decreased from 38 mm to 29 mm, representing a reduction of 23.7%. Upon assessment, the best overall efficacy reached SD.
5. Subject 111001: female, 47 years old, pathologically diagnosed with nasopharyngeal carcinoma, with lymph node metastasis. The target lesion was located in the left skull base. After 12 weeks of treatment with an anti-B7H3 antibody-drug conjugate in combination with serplulimab, the left skull base lesion remained stable at 45.5 mm from 45.4 mm. Upon assessment, the best overall efficacy reached SD.
6. Subject 101007: female, 59 years old, pathologically diagnosed with small cell lung cancer, with lymph node metastasis, brain metastasis, pleural metastasis, peritoneal metastasis. The target lesions were located in the inferior lobe of the left lung, the superior lobe of the left lung, the lymph nodes around the coeliac trunk, and the lymph nodes adjacent to the left gastric artery. After 12 weeks of treatment with an anti-B7H3 antibody-drug conjugate in combination with serplulimab, the lesion in the inferior lobe of the left lung decreased from 67 mm to 35 mm, representing a reduction of 47.8%; the lesion in the superior lobe of the left lung decreased from 19 mm to 8 mm, representing a reduction of 57.9%; the lymph node around the coeliac trunk decreased from 20 mm to 6 mm, representing a reduction of 70%; the lymph node adjacent to the left gastric artery decreased from 19 mm to 7 mm, representing a reduction of 63.1%. Upon assessment, the best overall efficacy reached confirmed PR.
7. Subject 102001: female, 57 years old, pathologically diagnosed with small cell lung cancer, with brain metastasis and pleural effusion. The target lesions were located in the hilum of the left lung and the inferior lobe of the left lung. After 6 weeks of treatment with an anti-B7H3 antibody-drug conjugate in combination with serplulimab, the lesion in the hilum of the left lung decreased from 30.1 mm to 24 mm, representing a reduction of 20.2%, and the lesion in the inferior lobe of the left lung decreased from 18 mm to 16 mm, representing a reduction of 11.1%. Upon assessment, the best overall efficacy reached SD.
8. Subject 108001: female, 48 years old, pathologically diagnosed with nasopharyngeal carcinoma, with liver metastasis. The target lesion was located in the right liver. After 6 weeks of treatment with an anti-B7H3 antibody-drug conjugate in combination with serplulimab, the lesion in the right liver decreased from 16 mm to 11 mm, representing a reduction of 31.2%. Upon assessment, the best overall efficacy reached PR.

In summary, the combined treatment regimen of the anti-B7H3 antibody-drug conjugate of the present invention and serplulimab preliminarily showed certain efficacy in patients with various advanced solid tumours, with gradual improvement in tumour conditions.

The above are merely preferred embodiments of the present invention and are not intended to limit the present invention. Any modifications, equivalent substitutions, improvements and the like made within the spirit and principles of the present invention shall fall within the scope of protection of the present invention. In addition, the technical solutions between the various embodiments may be combined with each other, but must be based on what is achievable by those of ordinary skill in the art; when the combination of the technical solutions is contradictory or cannot be achieved, such combination of the technical solutions should be considered as non-existent and not within the scope of protection claimed by the present invention.

## Claims

1. A method of treating cancer, the method comprising administering to a subject in need thereof an effective amount of:
(a) an anti-B7H3 antibody-drug conjugate, which comprises an anti-B7H3 antibody or antigen-binding fragment thereof and a camptothecin drug, wherein the anti-B7H3 antibody or antigen-binding fragment thereof comprises a heavy chain variable region and a light chain variable region, wherein:
the heavy chain variable region (VH) comprises:
(i) CDR-H1 comprising the amino acid sequence set forth in SEQ ID NO: 1;
(ii) CDR-H2 comprising the amino acid sequence set forth in SEQ ID NO: 2; and
(iii) CDR-H3 comprising the amino acid sequence set forth in SEQ ID NO: 3; and
the light chain variable region (VL) comprises:
(iv) CDR-L1 comprising the amino acid sequence set forth in SEQ ID NO: 4;
(v) CDR-L2 comprising the amino acid sequence set forth in SEQ ID NO: 5; and
(vi) CDR-L3 comprising the amino acid sequence set forth in SEQ ID NO: 6;
wherein the CDRs are determined in accordance with the Kabat definition scheme;
and (b) an additional therapeutic agent, wherein the additional therapeutic agent is selected from one or both of an anti-PD-1 antibody or antigen-binding fragment thereof and a chemotherapeutic drug.

2. The method of claim 1, wherein the heavy chain variable region comprises or is an amino acid sequence having at least 85% sequence identity to the amino acid sequence set forth in SEQ ID NO: 7, and the light chain variable region comprises or is an amino acid sequence having at least 85% sequence identity to the amino acid sequence set forth in SEQ ID NO: 8;
preferably, wherein the heavy chain variable region comprises or is the amino acid sequence set forth in SEQ ID NO: 7, and the light chain variable region comprises or is the amino acid sequence set forth in SEQ ID NO: 8;
more preferably, the anti-B7H3 antibody comprises a heavy chain with the amino acid sequence set forth in SEQ ID NO: 9 or with an amino acid sequence having at least 85% sequence identity to SEQ ID NO: 9, and a light chain with the amino acid sequence set forth in SEQ ID NO: 10 or with an amino acid sequence having at least 85% sequence identity to SEQ ID NO: 10;
still more preferably, the heavy chain of the anti-B7H3 antibody comprises the amino acid sequence set forth in SEQ ID NO: 9, and the light chain comprises the amino acid sequence set forth in SEQ ID NO: 10.

3. The method of claim 1 or 2, wherein the anti-B7H3 antibody or antigen-binding fragment thereof is the antibody designated as 2E3 or 2E3-02 in WO2022170971; more preferably, the anti-B7H3 antibody is the antibody designated as 2E3-02.

4. The method of any one of claims 1 to 3, wherein the method comprises administering to the subject in need thereof an effective amount of:
(a) an anti-B7H3 antibody-drug conjugate, wherein the anti-B7H3 antibody-drug conjugate is an antibody-drug conjugate having the structure of the following formula, a pharmaceutically acceptable salt, stereoisomer, or metabolite thereof, or a solvate thereof:
Tb-(L-D)q,
wherein:
Tb is an anti-B7H3 antibody or antigen-binding fragment thereof; preferably, Tb is as defined for the anti-B7H3 antibody or antigen-binding fragment thereof of any one of claims 1 to 3;
L is a linker and has the structure of formula: 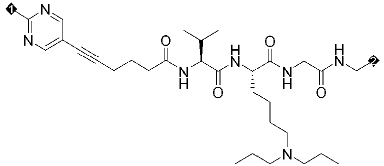
position 1 is linked to Tb, and position 2 is linked to D;
D is a bioactive molecular fragment, preferably a camptothecin drug;
q is selected from any numerical value ranging from 0.1 to 16.0; preferably, q is selected from 1, 2, 3, 4, 5, 6, 7, 8, 9 and 10; more preferably, q is 2, 4, 6 or 8;
and (b) an additional therapeutic agent, wherein the additional therapeutic agent is selected from one or both of an anti-PD-1 antibody or antigen-binding fragment thereof and a chemotherapeutic drug.

5. The method of claim 4, wherein the anti-B7H3 antibody-drug conjugate is an antibody-drug conjugate having the structure of formula I, a pharmaceutically acceptable salt, stereoisomer, or metabolite thereof, or a solvate of any of the foregoing: 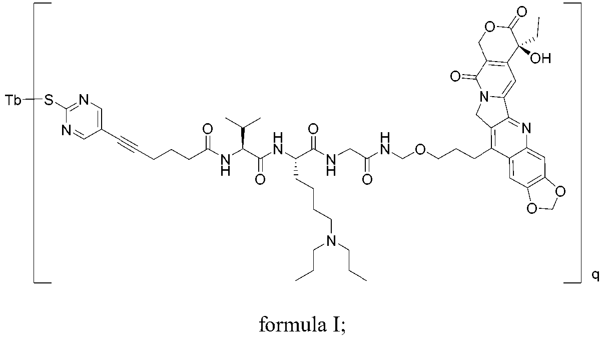 wherein S is a sulphur atom on Tb.

6. The method of any one of claims 1 to 5, wherein the anti-B7H3 antibody-drug conjugate is an antibody-drug conjugate having the structure of formula II, a pharmaceutically acceptable salt, stereoisomer, or metabolite thereof, or a solvate of any of the foregoing: 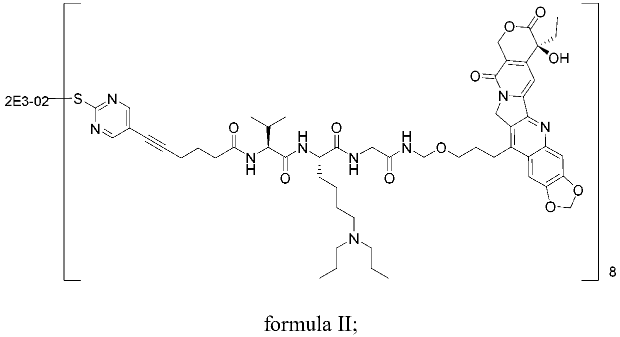
wherein S is a sulphur atom on Tb;
2E3-02 is an anti-B7H3 antibody.

7. The method of any one of claims 1 to 6, wherein the additional therapeutic agent is:
i. an anti-PD-1 antibody or antigen-binding fragment thereof;
ii. an anti-PD-1 antibody or antigen-binding fragment thereof and a chemotherapeutic drug.

8. The method of any one of claims 1 to 7, wherein the additional therapeutic agent satisfies one or both of the following conditions:
i. the anti-PD-1 antibody or antigen-binding fragment thereof being selected from one or more of AK103 (HX008), treprinumab, sintilimab, camrelizumab, tislelizumab, nivolumab (Opdivo), pembrolizumab (Keytruda), nofazinlimab, serplulimab (HLX10 or HANSIZHUANG), geptanolimab, lipustobart, BAT-1306, finotonlimab, rulonilimab, SG001, zimberelimab, spartalizumab, cemiplimab, and STI-A1110;
ii. the chemotherapeutic drug being selected from platinum-based drugs; preferably, the platinum-based drugs are selected from one or more of cisplatin, carboplatin, sulfatodiaminocyclohexane platin, nedaplatin, oxaliplatin, lobaplatin, satraplatin, miboplatin, enloplatin, iproplatin, and dicycloplatin.

9. The method of any one of claims 1 to 8, wherein the anti-B7H3 antibody-drug conjugate is administered to the subject at a dose ranging from about 0.1 mg/kg to about 15 mg/kg;
preferably, the anti-B7H3 antibody-drug conjugate is administered to the subject at a dose in the range of 0.1 to 10 mg/kg, 0.2 to 8 mg/kg, 0.3 to 6 mg/kg, 0.4 to 4 mg/kg, or 0.5 to 3 mg/kg of subject body weight;
more preferably, the anti-B7H3 antibody-drug conjugate is administered to the subject at a dose of 0.4 mg/kg, 0.5 mg/kg, 0.6 mg/kg, 0.8 mg/kg, 1.0 mg/kg, 1.2 mg/kg, 1.4 mg/kg, 1.6 mg/kg, 1.8 mg/kg, 2.0 mg/kg, 2.2 mg/kg, 2.4 mg/kg, 2.6 mg/kg, 2.8 mg/kg, 3.0 mg/kg, 4.0 mg/kg, or 5.0 mg/kg of subject body weight.

10. The method of any one of claims 1 to 9, wherein the anti-PD-1 antibody or antigen-binding fragment thereof is administered to the subject at a flat dose ranging from about 50 mg to about 650 mg, or at a dose of 1 to 10 mg/kg;
preferably, the anti-PD-1 antibody or antigen-binding fragment thereof is administered to the subject at a flat dose in the range of 100 to 600 mg, 100 to 500 mg, 100 to 400 mg, 200 to 600 mg, 200 to 500 mg, 200 to 400 mg, 150 to 450 mg, 150 to 250 mg, 250 to 350 mg, 350 to 450 mg, 450 to 550 mg, or 550 to 650 mg; more preferably, the anti-PD-1 antibody or antigen-binding fragment thereof is administered to the subject at a flat dose of 100 mg, 200 mg, 300 mg, 400 mg, 500 mg, or 600 mg;
still more preferably, the anti-PD-1 antibody or antigen-binding fragment thereof is administered to the subject at a dose of 1.0 mg/kg, 1.2 mg/kg, 1.4 mg/kg, 1.6 mg/kg, 1.8 mg/kg, 2.0 mg/kg, 2.4 mg/kg, 2.8 mg/kg, 3.0 mg/kg, 3.5 mg/kg, 4.0 mg/kg, 4.5 mg/kg, or 5.0 mg/kg of subject body weight.

11. The method of any one of claims 1 to 10, wherein the chemotherapeutic drug is a platinum-based drug, and the platinum-based drug is administered to the subject as follows:
i. at a dose in the range of about 50 to 150 mg/m²; preferably, the platinum-based drug is administered to the subject at a dose of 50 mg/m², 70 mg/m², 75 mg/m², 80 mg/m², 100 mg/m², 120 mg/m², or 150 mg/m²;
or
ii. at a dose calculated based on area under the curve (AUC) for each administration, wherein an injection rate of the dose of the administration is 1 to 10 mg/mL/min; preferably, the platinum-based drug is administered to the subject at an injection rate of 3 mg/mL/min, 4 mg/mL/min, 5 mg/mL/min, 6 mg/mL/min, or 7 mg/mL/min.

12. The method of any one of claims 1 to 11, wherein the anti-B7H3 antibody-drug conjugate, the anti-PD-1 antibody or antigen-binding fragment thereof, or the platinum-based drug is administered in a dosing cycle of 7 to 42 days, for example 21 to 42 days;
preferably:
the anti-B7H3 antibody-drug conjugate is administered at a frequency of once weekly, once every two weeks, once every three weeks, twice every three weeks, once every four weeks, once monthly, once every five weeks, or once every six weeks;
the anti-PD-1 antibody or antigen-binding fragment thereof is administered at a frequency of once weekly, once every two weeks, once every three weeks, once every four weeks, once monthly, once every five weeks, or once every six weeks;
the platinum-based drug is administered at a frequency of once weekly, once every two weeks, once every three weeks, once every four weeks, once monthly, once every five weeks, or once every six weeks.

13. The method of any one of claims 1 to 12, wherein the anti-B7H3 antibody-drug conjugate and the additional therapeutic agent are administered concurrently, separately, or sequentially.

14. The method of any one of claims 1 to 13, wherein the cancer is selected from a solid tumour; optionally, the cancer is selected from nasopharyngeal carcinoma, colorectal cancer, non-small cell lung cancer, small cell lung cancer, oesophageal squamous cell carcinoma, prostate cancer, and head and neck squamous cell carcinoma;
preferably, the cancer is selected from nasopharyngeal carcinoma, non-small cell lung cancer (NSCLC), and small cell lung cancer;
more preferably, the non-small cell lung cancer (NSCLC) is selected from squamous NSCLC, adenocarcinoma NSCLC, pulmonary lymphoepithelioma-like carcinoma, and large cell lung cancer; the nasopharyngeal carcinoma is selected from keratinising squamous cell carcinoma of the nasopharynx, non-keratinising nasopharyngeal carcinoma, basaloid squamous cell carcinoma of the nasopharynx, nasopharyngeal carcinoma in situ, and invasive nasopharyngeal carcinoma.

15. The method of claim 14, wherein the subject is a patient with locally recurrent or metastatic nasopharyngeal carcinoma who has previously failed treatment with a platinum-containing regimen; or the subject is a patient with locally recurrent or metastatic nasopharyngeal carcinoma who has not received systemic treatment.

16. The method of claim 14, wherein the subject is a patient with small cell lung cancer who has not received systemic treatment.

17. The method of claim 14, wherein the subject is a patient with locally advanced or metastatic non-small cell lung cancer who has previously failed treatment with a platinum-containing regimen and who is negative for epidermal growth factor receptor (EGFR) sensitising mutations, negative for anaplastic lymphoma kinase (ALK), and/or negative for c-ros proto-oncogene (ROS1); or the subject is a patient with locally advanced or metastatic non-small cell lung cancer who has not received systemic treatment and who is negative for EGFR sensitising mutations, negative for ALK, and/or negative for c-ros proto-oncogene (ROS1).

18. The method of any one of claims 1 to 17, wherein the route of administration of the anti-B7H3 antibody-drug conjugate or the additional therapeutic agent is oral administration, transdermal administration, intravenous injection, subcutaneous injection, or intramuscular injection; preferably, the route of administration of the anti-B7H3 antibody-drug conjugate or the additional therapeutic agent is intravenous injection.

19. The method of any one of claims 1 to 18, wherein one or more therapeutic effects in the subject are improved relative to baseline following administration of the anti-B7H3 antibody-drug conjugate and the anti-PD-1 antibody or antigen-binding fragment thereof, wherein the one or more therapeutic effects are selected from size of tumour derived from the cancer, objective response rate (ORR), depth of response (DpR), disease control rate (DCR), duration of response (DoR), time to response (TTR), progression-free survival (PFS), and overall survival (OS);
preferably, wherein the size of tumour derived from the cancer is reduced by at least about 10%, at least about 15%, at least about 20%, at least about 25%, at least about 30%, at least about 35%, at least about 40%, at least about 45%, at least about 50%, at least about 60%, at least about 70%, or at least about 80%, relative to the size of tumour prior to administration of the anti-B7H3 antibody-drug conjugate and the anti-PD-1 antibody or antigen-binding fragment thereof.

20. A pharmaceutical composition comprising an anti-B7H3 antibody-drug conjugate and an additional therapeutic agent, wherein the additional therapeutic agent is selected from one or both of an anti-PD-1 antibody or antigen-binding fragment thereof and a chemotherapeutic drug, and the anti-B7H3 antibody-drug conjugate, the anti-PD-1 antibody or antigen-binding fragment thereof, and the chemotherapeutic drug are as defined in any one of claims 1 to 19; preferably, the pharmaceutical composition is a pharmaceutical composition for treating cancer, and the cancer is as defined in claim 14;
optionally, the pharmaceutical composition further comprises one or more pharmaceutically acceptable excipients, diluents, or carriers.

21. A kit comprising an anti-B7H3 antibody-drug conjugate and an additional therapeutic agent, wherein the additional therapeutic agent is selected from one or both of an anti-PD-1 antibody or antigen-binding fragment thereof and a chemotherapeutic drug, and the anti-B7H3 antibody-drug conjugate, the anti-PD-1 antibody or antigen-binding fragment thereof, and the chemotherapeutic drug are as defined in any one of claims 1 to 19;
preferably, the kit comprises a container A and a container B, wherein the container A comprises the anti-B7H3 antibody-drug conjugate as an active ingredient, and the container B comprises the additional therapeutic agent as an active ingredient; and/or the kit is a kit for treating cancer, wherein the cancer is as defined in claim 14.
